(19) Europäisches Patentamt
European Patent Office
Office européen des brevets

(11) EP 1 232 269 B1

(12) **EUROPEAN PATENT SPECIFICATION**

(45) Date of publication and mention of the grant of the patent:
**04.02.2009 Bulletin 2009/06**

(21) Application number: **00981537.4**

(22) Date of filing: **24.11.2000**

(51) Int Cl.:
*C12N 9/04* (2006.01) *C12N 15/53* (2006.01)
*C12P 21/02* (2006.01) *C12N 1/06* (2006.01)

(86) International application number:
**PCT/IB2000/001886**

(87) International publication number:
**WO 2001/038544 (31.05.2001 Gazette 2001/22)**

(54) **METHOD FOR PURIFYING RECOMBINANT HEXOSE OXIDASE**

VERFAHREN ZUR REINIGUNG VON REKOMBINIERTEN HEXOSE OXIDASE

PROCEDE DE PURIFICATION DE HEXOSE OXYDASE DE RECOMBINAISON

(84) Designated Contracting States:
**AT BE CH CY DE DK ES FI FR GB GR IE IT LI LU MC NL PT SE TR**
Designated Extension States:
**AL LT LV MK RO SI**

(30) Priority: **24.11.1999 GB 9927801**

(43) Date of publication of application:
**21.08.2002 Bulletin 2002/34**

(60) Divisional application:
**08004919.0 / 1 944 373**

(73) Proprietor: **DANISCO A/S**
**1001 Copenhagen K. (DK)**

(72) Inventors:
- **JOHANSEN, Claus, Lindvald**
**DK-8270 Højbjerg (DK)**
- **KJAERULFF, Søren**
**DK-3400 Hillerød (DK)**
- **MADRID, Susan, Mampusti**
**DK-2950 Vedbaek (DK)**
- **PEDERSEN, Henrik**
**DK-8752 Østbirk (DK)**
- **POULSEN, Charlotte, Horsmans**
**DK-8220 Braband (DK)**
- **ZARGAHI, Masoud, Rajabi**
**DK-8230 Åbyhøj (DK)**

(74) Representative: **Harding, Charles Thomas**
**D Young & Co**
**120 Holborn**
**London EC1N 2DY (GB)**

(56) References cited:
**WO-A-96/26280** **WO-A-96/40935**
**WO-A-98/02559** **US-A- 4 346 018**
**US-A- 5 240 834** **US-A- 5 922 573**
**US-A- 5 977 306**

- **MIETZNER ET AL.: "Purification and characterization of the major iron-regulated protein expressed by pathogenic Neisseriae" JOURNAL OF EXPERIMENTAL MEDICINE, vol. 165, no. 4, 1 April 1987 (1987-04-01), pages 1041-1057, XP000946069**
- **JOSHI M. ET AL: 'Permeabilization of Yeast cells (Kluyveromyces fragilis) to lactose by cetyltrimethylammonium bromide' BIOTECHNOLOGY LETTERS vol. 9, no. 8, 1987, pages 549 - 554, XP008058724**
- **ALAMÄE T.; JÄRVISTE: 'Permeabilization of the methylotropic yeast Pichia pinu for intracellular enzyme analysis: a quantitative study' JOURNAL OF MICROBIOLOGICAL METHODS vol. 22, 1995, pages 193 - 205**
- **GOWDA L. ET AL: 'Permeabilization of Baker's yeast by cetyltrimethylammonium bromide for intracellular cenzyme catalysis' ENZYME MICROB TECHNOL vol. 13, February 1991, pages 154 - 157, XP009000717**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 232 269 B1

## Description

FIELD OF THE INVENTION

The present invention relates to a method for releasing an intracellular recombinant protein of interest (POI), in which the recombinant POI is a hexose oxidase enzyme.

In particular, the present invention relates to a method for releasing a soluble or membrane associated intracellular recombinant protein of interest (POI) using a membrane extracting composition which assists in the release of the POI, in which the recombinant POI is a hexose oxidase enzyme.

BACKGROUND OF THE INVENTION

The traditional way for recovering an intracellular POI, such as an enzyme, has been to use a mechanical disruption method (Naglak *et al* 1990) such as a bead mill or a cell homogenizer operating with a french press principle. However, these mechanical disruption methods suffer from the disadvantage that they are energy consuming methods with a low capacity and the cell homogenizers or similar equipment required for mechanical disruption are expensive to purchase. In addition, mechanical methods expose the cells, and hence the extracted POI to very harsh conditions, especially as most proteins will be denatured by the heat generated unless the mechanical device and/or homogenate is efficiently cooled. Furthermore, some cells, such as yeast cells (such as those from *Hansenula*) are difficult to disrupt mechanically and more than one passage through a cell homogenizer is needed. The cell homogenate may also contain cell wall fragments and DNA, which results in a high viscosity. This means that the separation of cell debris from the POI can prove to be a difficult operation. In addition, the resulting cell free homogenate may contain not only the intracellular POI but also a large number (sometimes several thousand) of different intracellular proteins and enzymes associated with the general cell metabolism. This means that the resultant cell free homogenate may be not only difficult to concentrate by ultrafiltration but may also provide problems with respect to obtaining the right commercial concentration of a given POI.

In order to minimise the potential detrimental effect of some mechanical disruption methods, chemical methods using, for example, detergents have been developed to permeabilize yeast cells. By way of example, the non-ionic detergent, polyethoxylated octylphenols, commercially available as Triton X-100, has been used either alone or in combination with freeze thaw cycles (referenced in Naglak *et al* 1990). In addition, US Patent No 5124256 (Crahay et al 1992) discloses a method whereby proteins were extracted from *Saccharomyces* yeast by means of treating the yeasts in an aqueous medium with a neutral water-soluble mineral salt and a non-ionic water-soluble polyethoxylated alkylphenol surfactant having a Hydrophilic Lipophilic Balance (HLB) of between 8 and 15. However, these non-ionic water-soluble polyethoxylated alkylphenol surfactants which include polyethoxylated octylphenols, nonylphenols and tributylphenols, (particularly those commercially available under the trade marks TritonX-100, Nonidet P-40 and Sapogenat T-080) suffer from the drawbacks that (i) they may not have a significant extracting effect when used alone and (ii) these surfactants can interfere with subsequent measurements of the enzymatic activity of the POI.

Several organic solvents have also been used to both permeabilize yeast cells in *in situ* enzymatic assays and for removing proteins from yeast cells. Examples of such solvents include but are not limited to toluene, ethyl acetate, dimethyl sulfoxide, and benzene combined with glycerol (Naglak *et al* 1990). However, these solvents are unattractive to use in industrial scale production when fermentor volumes of up to 200 $m^3$ are required.

Digitonin and other naturally occuring saponins have also been shown to permeabilize a number of eukaryotic cells (see Joshi *et al* 1989). Although the exact mechanism of digitonin permeabilization is not known, it is believed that digitonin forms a complex with the cholesterol present in the cell membrane and renders the membrane leaky. Digitonin permeabilization of yeast cells may also be due to the complexing of ergosterols of the yeast membrane. Joshi *et al* (1989) used digitonin (0.1%) to permeabilize the yeast *Kluyveromyces* which facilitated the intracellular catalysis of lactose to glucose and galactose. The non-ionic detergent saponin, from Quillaja Bark, is another cholesterol complexing agent, which is known to permeabilise at least mammalian cells (Naglak *et al* 1990). Again, like the non-ionic detergents as outlined above, the use of digitonin and other naturally occuring saponins may suffer from the drawback that when used alone, they may not have a significant extracting effect.

Chaotropic agents have also been used to faciliate the extraction of intracellular enzymes. By way of example, US Patent No 3801461 (Miyake and Shiosaka 1974) discloses a process for extracting intracellular enzymes produced in the mycelia or cells of fungi or bacteria using a chaotrophic solution such as a urea solution. US Patent No 4683293 (Craig 1987) also discloses a method for selective extraction of lipophilic proteins from transformed cells of the genus *Pichia* by cell lysis in the presence of chaotrophic salts such as sodium thiocyanate, sodium iodide, sodium hypochlorite, lithium bromide, guanidium hydrochloride and urea. However, chaotrophic agents suffer from the disadvantage that exposure of the POI to a chaotrophic agent, such as urea can result in an actual loss of enzyme activity through denaturation of the POI.

Gowda et al (1991; Enzyme Microb. Technol 13: 154-157) describe the premeabilisation of *Saccharomyces cerevisiae* cells by CTAB and that the activity of the intracellular enzymes ADH, G6PDH and hexokinase was significantly higher in permeabilised cells.

Alamäe and Järviste (1995; Journal of Microbiological Methods 22:193-205) describe the permeabilisation of the methylotrophic yeast *Pichia pinus* with surfactant, digitonin or CTAB and measure the activities of the intracellular enzymes alcohol oxidase, formaldehyde dehydrogenase and formate dehydrogenase.

In addition to the drawbacks cited above, the above cited prior art only relates to the permeabilisation of host cells to low molecular weight molecules while the POI remains unchanged within the cell. In particular, none of the above cited prior art relates to the extraction of a membrane associated intracellular POI under conditions which do not affect the nature and/or activity of the POI. More in particular, none of the above cited prior art relates to a method for assisting in the release of a membrane associated intracellular POI which is trapped and is incapable of being secreted from a host cell.

The present invention thus seeks to overcome the problems associated with the extraction methods of the prior art.

The present invention thus provides a method for releasing a soluble or membrane associated intracellular recombinant protein of interest (POI) from a host organism, in which the recombinant POI is a hexose oxidase enzyme.

SUMMARY

There is mentioned herein a method for assisting in the release of a soluble or membrane associated intracellular POI which is trapped and/or is incapable of being secreted from a host cell. The extraction of an intracellular POI using the method described herein was compared with a traditional cell disruption method and with extraction procedures using other ionic/non ionic detergents and emulsifiers. Combinations of detergents with protease and salts were also investigated. The results indicate that the extraction of a soluble or membrane associated intracellular POI using the method mentioned herein is advantageous because:

(i) traditional cell disruption techniques can be avoided;
(ii) the intracellular POI may be recovered free from contaminating DNA and cell wall fragments;
(iii) the intracellular POI may be recovered from a eukaryotic host organism, such as yeast, before glycosylation takes place. Overglycosylation of secreted proteins is a well known problem especially in eukaryotic host organisms such as yeast. This drawback associated with yeast expression systems has led to a reluctance to use yeast as a production system even though yeast expression vectors are capable of producing proteins at high levels of expression with a large amounts of biomass, and additionally, yeast has approved use in food. By expressing the POI intracellularly and then extracting the POI with the method mentioned herein, the POI will be non-glycosylated, because the POI has not passed through the secretion pathway where glycosylation takes place;
(iv) the fermentation procedure that precedes the method mentioned herein can be carried out at any pH that is suitable for the host cell. It is well known in the art that a secreted POI may be affected by the pH of its extracellular growth medium. Up until now, it was often necessary to maintain the pH of a host organism growth medium at an approximately neutral pH because fermentations at such a pH were deemed necessary to maintain the stability of a secreted POI even though they usually increased the risk of bacterial contamination. With the method mentioned herein, the POI is not secreted. Thus, the pH of the host organism growth medium is irrelevant as the
(i) intracellular pH remains constant irrespective of the media pH. Accordingly, this permits the growth of a host organism (such as yeast) at a lower pH (such as pH 4.0) which reduces the risk of bacterial contamination without affecting either biomass or POI production; and
(ii) the method described herein can be used to prevent contact of the intracellular POI with the extracellular growth medium. This is advantageous if the POI is unstable in the extracellular media, because of, for example, protease sensitivity. By expressing the protein intracellularly and then extracting with the method described herein contact with the extracellular media is avoided.

SUMMARY ASPECTS

In one broad aspect, there is mentioned herein a method for releasing a protein of interest (POI) from a cell. The method comprises the steps of: providing a cell comprising a soluble or membrane associated intracellular POI; contacting the cell with a membrane extracting composition; and causing the POI to be released from the cell under conditions sufficient for the release of the POI and in a soluble form. Here, the POI may be an intracellular protein of interest and/or the POI may be an hexose oxidase (D-hexose: $O_2$-oxidoreductase, EC 1.1.3.5).

DETAILED ASPECTS

The present invention provides, in one aspect, a method for extracting a soluble or membrane associated intracellular recombinant protein of interest (POI) from a bacterial, yeast or fungal cell comprising the steps of:

(a) providing a bacterial, yeast or fungal cell comprising a soluble or membrane associated intracellular recombinant POI;
(b) releasing the recombinant POI from the cell by contacting the cell with a membrane extracting composition comprising a quaternary ammonium compound at a concentration of between 0.05% to 0.6% by weight, under conditions sufficient for the release of the recombinant POI and in a soluble form; and
(c) recovering the recombinant POI from the membrane extracting composition;

in which the recombinant POI is a hexose oxidase (HOX) enzyme.

In another aspect, the present invention provides a method for screening for mutated cells or transformed cells producing elevated levels of a soluble or membrane associated intracellular recombinant POI comprising the steps of:

(a) growing the mutated cells at 30˚C;
(b) incubating the mutated cells or transformed cells with the membrane extracting composition in a method as defined in any preceding claim;
(c) recovering the cell free medium;
(d) screening the cell free medium for elevated levels of the intracellular recombinant POI;

such that the presence of the intracellular recombinant POI in the cell free medium is indicative that the intracellular recombinant POI has been released, in which the mutated or transformed cell is a bacterial, yeast or fungal cell and in which the recombinant POI is a hexose oxidase (HOX) enzyme.

In a further aspect, the present invention provides the use of a membrane extracting composition comprising a quaternary ammonium compound to extracting a soluble or membrane associated intracellular recombinant POI from a bacterial, yeast or fungal cell, in which the membrane extracting composition comprises a quaternary ammonium compound at a concentration of between 0.05% to 0.6% by weight and is contacted with the bacterial, yeast or fungal cell, in which the released recombinant POI is recovered from the membrane extracting composition and in which the recombinant POI is a hexose oxidase (HOX) enzyme.

According to one aspect, there is provided a method for releasing a soluble or membrane associated intracellular protein of interest (POI) from a transformed cell comprising the steps of: providing a transformed cell comprising an soluble or membrane associated intracellular POI; contacting the transformed cell with a membrane extracting composition; and causing the POI to be released from the transformed cell under conditions sufficient for the specific release of the POI and in a soluble form.

According to another aspect, there is provided a method for releasing a HOX enzyme from a transformed cell comprising the steps of: providing a transformed cell comprising a HOX enzyme; contacting the transformed cell with a membrane extracting composition; and causing the HOX enzyme to be released from the transformed cell under conditions sufficient for the specific release of the a HOX enzyme and in a soluble form.

According to another aspect, there is provided a method for releasing an interleukin 1 receptor antagonist (IL-lra) from a transformed cell comprising the steps of: providing a transformed cell comprising a IL-lra; contacting the transformed cell with a membrane extracting composition; and causing the IL-lra to be released from the transformed cell under conditions sufficient for the specific release of the IL-lra and in a soluble form.

According to another aspect, there is provided a method for screening for mutants producing elevated levels of a soluble or membrane associated intracellular POI comprising the steps of: growing the mutated cells at 30˚C; incubating the mutated cells with the membrane extracting composition; recovering the cell free medium; screening the cell free medium for elevated levels of the intracellular POI; such that the presence of the intracellular POI cell free medium is indicative that the intracellular POI has been released.

According to another aspect, there is provided a membrane extracting composition suitable for releasing a soluble or membrane associated intracellular POI wherein the composition is contacted with the cell under the following conditions of: a percentage by weight of quarternary ammonium compound from about 0.05% to about 0.6% (more especially from about 0.1% to about 0.5%, more especially from about 0.2% to about 0.45%, more especially about 0.4%); and a pH optima of from about 2.0 to about 11.0 (more especially from about 5.0 to about 7.0, more especially about 6.3); a temperature optima of from about 4˚C to about 40˚C, (more especially from about 20 ˚C to about 30˚C, more especially about 25˚C).

According to another aspect, there is provided a membrane extracting composition comprising a quaternary ammonium compound suitable for releasing a soluble or membrane associated intracellular POI.

According to another aspect, there is provided a substantially non-glycosylated POI released from a eukaryotic host organism.

Other aspects and advantages are presented in the accompanying claims and in the following description and discussion. These aspects are presented under separate section headings. However, it is to be understood that the teachings under each section heading are not necessarily limited to that particular section heading.

DETAILED DESCRIPTION

The present invention is based on the highly surprising finding that a membrane extraction composition comprising quaternary ammonium compounds may be used to obtain a fast, specific and economically efficient extraction of a soluble or membrane associated intracellular POI, without resorting to the use of traditional cell disruption techniques. Advantageously and unexpectedly, the resulting cell extract contains very little contaminating intracellular DNA and is relatively free of cell wall fragments thereby simplifying any further purification steps to which the POI may be subjected. This is in contradistinction to the prior art mechanical extraction methods.

INTRACELLULAR PROTEIN

As used herein, the term "intracellular" POI means a POI which is found within or inside a cell or cells. The intracellular POI may be localised within a cell (such as in the cytoplasm of the cell) even though it has a signal secretory mechanism. In this regard, the intracellular POI may be a POI which is not actively secreted from a cell or is incapable of being secreted by the cell even though it has a signal sequence secretory mechanism. In the alternative, the intracellular POI may be a naturally secreted POI which has been engineered to prevent its secretion from a cell. Alternatively, the POI may be a chimeric protein comprising a membrane binding domain.

The method described herein is in contrast to the method described in Ahlstrom and Edebo ((1994) PEMS Microbiology Letters 119 7-12) who report on the release of the periplasmic β-lactamase from *E. coli* with tetradecyl betainate. The periplasm is the region in a bacterial cell between the cell membrane and the cell wall. Thus, the periplasmic β-lactamase from *E. Coli* is localised outside the cell membrane and is not a cytoplasmic enzyme. In contradistinction, the POI mentioned herein is an intracellular POI which is found within or inside a cell or cells.

MEMBRANE ASSOCIATED POI

As used herein, the term "membrane associated POI" means a POI which may be localised in the proximity of, but may not be substantially associated with a cell or plasma membrane. Thus, the membrane associated enzyme is not a substantially membrane bound protein or the membrane associated enzyme is not substantially bound to a cell membrane. The membrane associated POI may be solubilised by a mechanical treatment with a cell homogeniser.

MEMBRANE BOUND POI

As used herein, the term "membrane bound POI" means a protein which is not rendered soluble by mechanical treatment with a cell homogeniser.

SPECIFIC RELEASE

The term "specific release" means that the specific activity of the POI is higher than when it has been extracted by mechanical means - such as by use of a bead mill or a cell homogenizer operating with a french press principle.

TRANSFORMED CELL

The term "transformed cell" includes cells that have been transformed by use of recombinant DNA techniques. The transformation typically occurs by insertion of one or more nucleotide sequences into a cell that is to be transformed. The inserted nucleotide sequence may be a heterologous nucleotide sequence (i.e. is a sequence that is not natural to the cell that is to be transformed. In addition, or in the alternative, the inserted nucleotide sequence may be an homologous nucleotide sequence (i.e. is a sequence that is natural to the cell that is to be transformed) - so that the cell receives one or more extra copies of a nucleotide sequence already present in it.

MEMBRANE EXTRACTING COMPOSITION

As used herein, the term "membrane extracting composition" means a composition capable of affecting components in a cellular membrane such that a membrane bound and/or membrane associated intracellular POI is sufficiently dissociated and/or released from the membrane component and the POI is easily recovered and/or harvested from the membrane extracting composition. The POI may also be a soluble POI. In a highly preferred embodiment, the membrane extracting composition used herein comprises one or more quaternary ammonium compounds or combinations thereof.

QUARTERNARY AMMONIUM COMPOUNDS

As used herein, the term "quaternary ammonium compound" means a compound derivable from ammonium hydroxide or an ammonium salt by replacement of all four hydrogen atoms of the $NH_4^+$ ion by organic groups which may be the same or different. Typically one of the organic groups is a long chain ($C_8$-$C_{18}$) alkyl group and the other three are shorter chain alkyl or other groups.

In a preferred embodiment, these compounds have the structure:

$$CH_3\text{-}(CH_2)_n\text{-}N(CH_3)^+{}_3$$

where n is the number of methylene groups in the chain and where the counter ion may be a halogen such as a chloride or bromide ion. These compounds have the properties of cationic detergents and are powerful antimicrobial agents.

Examples of these quaternary ammonium compound include but is not limited to Lauroyl Trimethyl Ammonium Bromide (LTAB), Myristyl Trimethyl Ammonium Chloride (MTAC), Cetyl Trimethyl Ammonium Chloride (CTAC), Cetyl Trimethyl Ammonium Bromide (CTAB), Cetrimide (or Cetrimidum which comprises a mixture of alkylammonium bromides, principally CTAB), Stearoyl Trimethyl Ammonium Chloride (STAC), Stearoyl Trimethyl Ammonium Bromide (STAB), Benzalkonium Chloride (alkyldimethylbenzylammonium chloride), N-Cetylpyridinium Bromide (N-Hexadecylpyridinium bromide), N-Cetylpyridinium Chloride (N-Hexadecylpyridinium chloride), Benzyl Dimethyl Tetradecyl Ammonium Chloride, and Benzyl Dimethyl Hexadecyl Ammonium Chloride.

By way of example, the structure of some of these compounds is illustrated as follows. The compounds are listed in the order of increasing methylene groups:

LTAB is $H_3C\text{-}(CH_2)_{11}\text{-}N\ (CH_3)_3Br$

MTAC is $H_3C\text{-}(CH_2)_{13}\text{-}N\ (CH_3)_3Cl$

CTAC is $H_3C\text{-}(CH_2)_{15}\text{-}N\ (CH_3)_3Cl$

CTAB is $H_3C\text{-}(CH_2)_{15}\text{-}N\ (CH_3)_3Br$

STAC is $H_3C\text{-}(CH_2)_{17}\text{-}N\ (CH_3)_3Cl$

STAB is $H_3C\text{-}(CH_2)_{17}\text{-}N\ (CH_3)_3Br$

Preferably the quaternary ammonium compound is cetylpyridinium chloride (CPC, $C_{21}H_{38}NCl$). The structure of CPC is illustrated as follows:

$C_{21}H_{38}NCl$

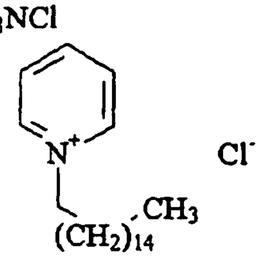

Preferably the quaternary ammonium compound is cetylpyridinium bromide (CPB, $C_{21}H_{38}NBr$). The structure of CPB is illustrated as follows:

$C_{21}H_{38}NBr$

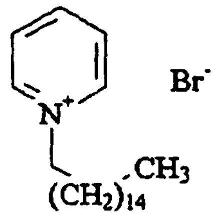

Preferably the quaternary ammonium compound is Benzyl Dimethyl Tetradecyl Ammonium Chloride (BDTAC: $C_{23}H_{42}NCl$). The structure of BDTAC is illustrated as follows:

$C_{23}H_{42}NCl$

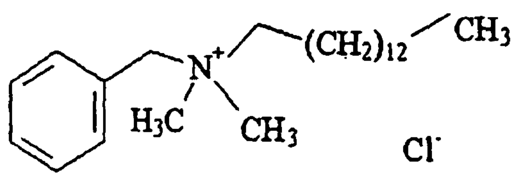

Preferably the quaternary ammonium compound is Benzyl Dimethyl Hexadecyl Ammonium Chloride (BDHAC: $C_{25}R_{46}NCl$). The structure of BDHAC is illustrated as follows:

$C_{25}H_{46}NCl$

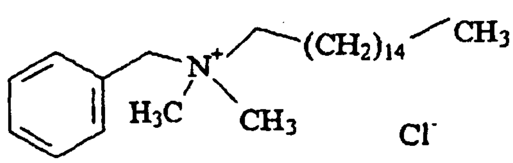

$C_{23}H_{42}NCl$

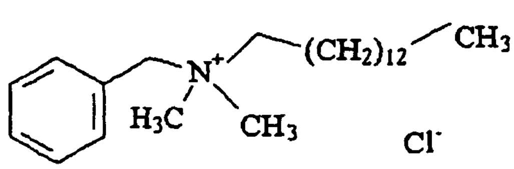

Preferably the quaternary ammonium compound is benzalkonium chloride (alkyldimethylbenzylammonium chloride).

The structure of benzalkonium chloride is illustrated as follows:

$C_{12}H_{25}NOH_3)_2C_7H_7Cl$

A comparison of the structure of CTAB and benzalkonium chloride (also known as Alkyldimethylbenzylammonium chloride - hereinafter referred to under the proprietary name of Rodalon) is illustrated as follows:

**Rodalon:**

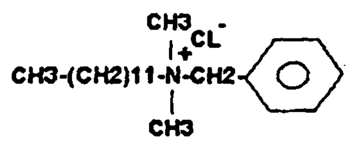

**CTAB:**

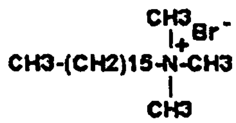

Preferably the quaternary ammonium compound is Lauroyl Trimethyl Ammonium Bromide (LTAB).

Preferably the quaternary ammonium compound is Cetyl Trimethyl Ammonium Chloride (CTAC).

Preferably the quaternary ammonium compound is Cetyl Trimethyl Ammonium Bromide (CTAB).

The cationic detergent CTAB has been shown to be capable of altering yeast permeability, probably by causing the formation of transmembrane pores, similar to the suggested mechanism for two other non-ionic detergents such as Pluronic F-68 and Triton X-100 (King *et al* 1991). The alteration of cellular permeability using detergents such as CTAB has facilitated the measurement of intracellular enzyme activities in whole cells (Sekhar *et al* 1999). Moreover, the development of CTAB permeabilised cells has proved useful for intracellular enzyme catalysis in, for example, cells from yeast strains such as *Saccharomyces cerevisiae* (Gowda *et al* 1991) and *Kluyveromyces fragilis* (Joshi *et al* 1987). In these studies, it is important to note that the detergent CTAB made yeast cells permeable to low molecular weight molecules (such as substrates, products, cofactors), while intracellular enzymes and other POIs remained unchanged within the cell. In contradistinction to the method described herein, none of the above mentioned studies has disclosed or suggested that the detergent CTAB (or related quarternary ammonium compounds such as LTAB or CTAC) may be used to assist in the release of a soluble or membrane associated intracellular POI from a host cell.

The cationic detergent CTAB has also been commonly used in methods for isolating DNA/RNA molecules. By way of example, DNA molecules may be isolated by treating cells with CTAB at high temperatures (about 65˚C) and a low salt concentrations (less than 0.6M NaCl) such that a DNA-CTAB precipitate is formed and easily recovered. The CTAB detergent is also frequently used to extract nucleic acids from plants where coprecipitation of neutral polysaccharides, by either ethanol or isopropanol, may pose a major problem. CTAB has also been used in the direct lysis and precipitation of the DNA from the supernatant of *E. coli* cultures infected with filamentous phage (see Ishaq et al 1990 Biotechniques 9(1): 19-20, 22, 24; Kambouris et al 1999: FEMS Immunol Med Microbiol 25(3): 255-64; Kuipers et al 1999 Ann Rheum Dis 58(2): 103-8; Velegraki et al 1999 Med Mycol 37(1) 69-73; White et al 1998 Med Mycol 36(5): 299-303; Woodhead et al 1998 Mol Biotechnol 9(3): 243-6; Mito and Detschart 1998 Parasitol Res 84(7) 596-7; Zhang et al 191998) J Virol Methods 71(1) 45-50; Reineke et al (1998) Insect Mol Biol 7(1) 95-9). All of these CTAB based methods for the isolation of DNA molecules rely on the exploitation of the properties of CTAB to precipitate nucleic acids and acid polysaccharides while maintaining the remaining proteins and neutral polysaccharides in solution. Surprisingly and unexpectedly, the method described herein facilitates not only the precipitation but also the retention of intracellular DNA. Consequently, the method described herein facilitates the selective release of an intracellular POI.

RELEASING

According to the method mentioned herein, the soluble or membrane associated intracellular POI is released from a host cell or cells by contacting the cells with a membrane extracting composition under conditions sufficient for the release of the intracellular POI.

PREFERABLE CONDITIONS SUFFICIENT FOR RELEASING THE POI

(I) % QUATERNARY AMMONIUM COMPOUND

Preferably the membrane extracting composition comprises from about 0.05% to about 0.6% by weight of a quaternary ammonium compound, preferably from about 0.1% to about 0.5% by weight of a quaternary ammonium compound, preferably from about 0.2% to about 0.45 % by weight of a quaternary ammonium compound, more preferably about 0.4% by weight of a quaternary ammonium compound.

Preferably the quaternary ammonium compound is LTAB.

Preferably the quaternary ammonium compound is CTAC.

Preferably the quaternary ammonium compound is CTAB.

Preferably the quaternary ammonium compound is Benzalkonium Chloride ($C_{12}H_{25}N\ (CH_3)_2C_7H_7Cl$).

Preferably the quaternary ammonium compound is Cetylpyridinium Chloride (CPC, $C_{21}H_{38}NCl$).

Preferably the quaternary ammonium compound is Cetylpyridinium Bromide (CPB, $C_{21}H_{38}NBr$).

Preferably the quaternary ammonium compound is Benzyl Dimethyl Tetradecyl Ammonium Chloride (BDTAC: $C_{23}H_{42}NCl$).

Preferably the quaternary ammonium compound is Benzyl Dimethyl Hexadecyl Ammonium Chloride (BDTAC: $C_{25}H_{46}NCl$).

(II) TEMPERATURE

Preferably the host cell is contacted with the membrane extracting composition at temperatures from about 4˚C to about 40˚C.

Preferably the host cell is contacted with the membrane extracting composition at temperatures from about 20˚C to about 30˚C.

Preferably the host cell is contacted with the membrane extracting composition at temperatures of about 25˚C.

Preferably the above temperature ranges are higher if the POI is a thermostable POI.

(III) pH

Preferably the host cell is contacted with the membrane extracting composition at a pH of from about 2.0 to about 11.0.

Preferably the host cell is contacted with the membrane extracting composition at a pH of from about 5.0 to about 7.0.

Preferably the host cell is contacted with the membrane extracting composition at a pH of about 6.3.

It is highly advantageous that the fermentation procedure that precedes the method described herein can be carried out at any pH that is suitable for the host cell. It is well known in the art that a secreted POI may be affected by the pH of its extracellular growth medium. Up until now, it was often necessary to maintain the pH of a host organism growth medium at an approximately neutral pH because fermentations at such a pH were deemed necessary to maintain the stability of a secreted POI even though they usually increased the risk of bacterial contamination. With the method described herein, the POI is not secreted. Thus, the pH of the host organism growth medium is irrelevant as the intracellular pH remains constant irrespective of the media pH. Accordingly, the method mentioned herein permits the growth of a host organism (such as yeast) at a lower pH (such as pH 4.0) which reduces the risk of bacterial contamination without affecting either biomass or POI production.

A further advantage of the method mentioned herein is that it can be used to prevent contact of the intracellular POI with the extracellular growth medium. This is advantageous if the POI is unstable in the extracellular media, because of, for example, protease sensitivity. By expressing the protein intracellularly and then extracting with the method described herein contact with the extracellular media is avoided.

POI RECOVERY

The intracellular POI which has been extracted in accordance with the method described herein may be further treated by employing techniques known by those of skill in the art to further concentrate and purify the POI. Thus, the extracted intracellular POI may be concentrated by for example, ultrafiltration, passage through a reverse phase resin followed by elution with a minimum volume of solvent, precipitation, ultrafiltration and lyophilization. Techniques available for further purification of the POI include but are not limited to size fractionation employing size exclusion resins, high performance liquid chromatography, ion exchange and hydrophobic chromatography.

POI

As used herein, the term "POI" includes but is not limited to, a protein, polypeptide or peptide including, but not limited to, a structural protein, an enzyme, a cytokine (such as an interferon and/or an interleukin), an interleukin receptor antagonist (such as IL-1ra), an antibiotic, a polyclonal or monoclonal antibody, or an effective part thereof, such as an Fv fragment, which antibody or part thereof may be natural, synthetic or humanized, a peptide hormone, an antigen (such as a bacterial/viral/protozoal/parasitic antigen), a tumour antigen, a receptor, a ligand, a regulatory factor, a signalling molecule, a neurotransmitter, a clotting factor, or any other protein including but not limited to a membrane bound protein and/or a membrane associated protein.

In the method described herein, the POI is expressed intracellularly, that is, it is an intracellular POI.

The POI may be produced by recombinant DNA techniques using a nucleotide sequence of interest (NOI).

NOI

As used herein, the term "NOI" is defined to encompass DNA and RNA of both synthetic and natural origin which DNA or RNA may contain modified or unmodified deoxy- or dideoxy- nucleotides or ribonucleotides or analogs thereof. The nucleic acid may exist as single- or double-stranded DNA or RNA, an RNA/DNA heteroduplex or an RNA/DNA copolymer, wherein the term "copolymer" refers to a single nucleic acid strand that comprises both ribonucleotides and deoxyribonucleotides. The NOI may even be codon optimised to further increase expression.

SYNTHETIC

The term "synthetic", as used herein, is defined as that which is produced by *in vitro* chemical or enzymatic synthesis. It includes but is not limited to NOIs made with optimal codon usage for host organisms such as the methylotrophic yeasts *Pichia* and *Hansenula.*

CONSTRUCTS

The NOI may be operatively linked to transcriptional and translational regulatory elements active in a host cell of interest. The NOI may also encode a fusion protein comprising signal sequences such as, for example, those derived from the glucoamylase gene from *Schwanniomyces occidentalis,* α-factor mating type gene from *Saccharomyces cerevisiae* and the TAKA-amylase from *Aspergillus oryzae.* Alternatively, the NOI may encode a fusion protein comprising a membrane binding domain.

EXPRESSION VECTOR

The NOI may be expressed at the desired levels in a host organism using an expression vector.

An expression vector comprising the NOI mentioned herein can be any vector which is capable of expressing the gene encoding the NOI in the selected host organism, and the choice of vector will depend on the host cell into which it is to be introduced. Thus, the vector can be an autonomously replicating vector, i.e. a vector that exists as an episomal entity, the replication of which is independent of chromosomal replication, such as, for example, a plasmid, a bacteriophage or an episomal element, a minichromosome or an artificial chromosome. Alternatively, the vector mentioned herein is one which, when introduced into a host cell, is integrated into the host cell genome and replicated together with the chromosome.

COMPONENTS OF THE EXPRESSION VECTOR

The expression vector typically includes the components of a cloning vector, such as, for example, an element that permits autonomous replication of the vector in the selected host organism and one or more phenotypically detectable markers for selection purposes. The expression vector normally comprises control nucleotide sequences encoding a promoter, operator, ribosome binding site, translation initiation signal and optionally, a repressor gene or one or more activator genes. Additionally, the expression vector may comprise a sequence coding for an amino acid sequence capable of targeting the POI to a host cell organelle such as a peroxisome or to a particular host cell compartment. Such a targeting sequence includes but is not limited to the sequence SKL. In the present context, the term "expression signal" includes any of the above control sequences, repressor or activator sequences. For expression under the direction of control sequences, the NOI encoding the POI is operably linked to the control sequences in proper manner with respect to expression.

PROMOTER

In the vector, the NOI encoding for the POI is operably combined with a suitable promoter sequence. The promoter can be any DNA sequence having transcription activity in the host organism of choice and can be derived from genes that are homologous or heterologous to the host organism.

BACTERIAL PROMOTERS

Examples of suitable promoters for directing the transcription of the modified nucleotide sequence described herein in a bacterial host include the promoter of the *lac* operon of E. *coli,* the *Streptomyces coelicolor* agarase gene *dagA* promoters, the promoters of the *Bacillus licheniformis* α-amylase gene *(amyL),* the promoters of the *Bacillus stearothermophilus* maltogenic amylase gene *(amyM),* the promoters of the *Bacillus amyloliquefaciens* α-amylase gene *(amyQ),* the promoters of the *Bacillus subtilis xylA and xylB* genes and a promoter derived from a *Lactococcus* sp.-derived promoter including the P170 promoter. When the gene encoding the POI mentioned herein is expressed in a bacterial species such as *E. coli, a* suitable promoter can be selected, for example, from a bacteriophage promoter including a T7 promoter and a phage lambda promoter.

FUNGAL PROMOTERS

For transcription in a fungal species, examples of useful promoters are those derived from the genes encoding the, *Aspergillus oryzae* TAKA amylase, *Rhizomucor miehei* aspartic proteinase, *Aspergillus niger* neutral α-amylase, *A. niger* acid stable α-amylase, *A. niger glucoamylase, Rhizomucor miehei lipase, Aspergillus oryzae* alkaline protease, *Aspergillus oryzae* triose phosphate isomerase or *Aspergillus nidulans* acetamidase.

YEAST PROMOTERS

Examples of suitable promoters for the expression in a yeast species include but are not limited to the Gal 1 and Gal 10 promoters of *Saccharomyces cerevisiae* and the *Pichia pastoris AOX1 or* AOX2 promoters.

HOST ORGANISMS

(I) BACTERIAL HOST ORGANISMS

Examples of suitable bacterial host organisms are gram positive bacterial species such *as Bacillaceae including Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus lautus, Bacillus megaterium and Bacillus thuringiensis, Streptomyces* species such as *Streptomyces murinus,* lactic acid bacterial species including *Lactococcus* spp. such as *Lactococcus lactis, Lactobacillus* spp. including *Lactobacillus reuteri, Leuconostoc spp., Pediococcus* spp. and *Streptococcus spp.* Alternatively, strains of a gram-negative bacterial species belonging to *Enterobacteriaceae* including *E. coli,* or to *Pseudomonadaceae* can be selected as the host organism.

(II) YEAST HOST ORGANISMS

A suitable yeast host organism can be selected from the biotechnologically relevant yeasts species such as but not limited to yeast species such as *Pichia sp., Hansenula sp or Kluyveromyces, Yarrowinia* species or a species of *Saccharomyces including Saccharomyces cerevisiae* or a species belonging to *Schizosaccharomyce* such as, for example, *S. Pombe* species.

Preferably a strain of the methylotrophic yeast species *Pichia pastoris* is used as the host organism.

Preferably the host organism is a *Hansenula* species.

It is highly advantageous to use the method described herein to recover an intracellular POI from a eukaryotic host organism, such as yeast, before glycosylation takes place. Overglycosylation of secreted proteins is a well known problem especially in eukaryotic host organisms such as yeast. This drawback associated with yeast expression systems has led to a reluctance to use yeast as a production system even though yeast expression vectors are capable of producing proteins at high levels of expression with a large amounts of biomass, and additionally, yeast has approved use in food. By expressing the POI intracellularly and then extracting the POI with the method described herein, the POI will be non-glycosylated, because the POI has not passed through the secretion pathway where glycosylation takes place.

(III) FUNGAL HOST ORGANISMS

Suitable host organisms among filamentous fungi include species of *Aspergillus, e.g. Aspergillus niger, Aspergillus oryzae, Aspergillus tubigensis, Aspergillus awamori or Aspergillus nidulans.* Alternatively, strains of a *Fusarium* species, e.g. *Fusarium oxysporum* or of a *Rhizomucor* species such as *Rhizomucor miehei* can be used as the host organism. Other suitable strains include *Thermomyces* and *Mucor* species.

LARGE SCALE APPLICATION

In one preferred embodiment, the POI is used for large scale applications.

Preferably the POI is produced in a quantity of from 1 g per litre to about 2g per litre of the total cell culture volume after cultivation of the host organism.

Preferably the POI is produced in a quantity of from 100mg per litre to about 900mg per litre of the total cell culture volume after cultivation of the host organism.

Preferably the POI is produced in a quantity of from 250mg per litre to about 500mg per litre of the total cell culture volume after cultivation of the host organism.

FOOD APPLICATIONS

In one preferred embodiment, the method described herein is used to release a POI for use in the manufacture of food products, such as beverages.

In another preferred embodiment, the method described herein is used to release a POI for use in the preparation of detergents.

In another preferred embodiment, the method described herein is used to release a POI suitable for use in baking.

In another preferred embodiment, the method described herein is used to release a POI suitable for use as a dough improving agent.

In another preferred embodiment, the method described herein is used to release a POI suitable for improving the properties of a flour dough, a flour dough improving composition and improved food products (see WO 96/39851 and EP-B-0 833 563).

In a preferred embodiment, the released POI is a hexose oxidase (D-hexose: $O_2$-oxidoreductase, EC 1.1.3.5).

HOX ENZYME

Hexose oxidase (D-hexose: $O_2$-oxidoreductase, EC 1.1.3.5) (also referred to as HOX) is an enzyme that in the presence of oxygen is capable of oxidising D-glucose and several other reducing sugars including maltose, lactose and cellobiose to their corresponding lactones with subsequent hydrolysis to the respective aldobionic acids. Accordingly, HOX differs from another oxidoreductase, glucose oxidase, which can only convert D-glucose, in that the enzyme can utilise a broader range of sugar substrates. The oxidation catalysed by HOX can be illustrated as follows:

D-glucose + $O_2$ ------> γ-D-gluconolactone + $H_2O_2$,

or

D-galactose + $O_2$ -----> γ-D-galactonolactone + $H_2O_2$

HOX is produced naturally by several marine algal species. Such species are found *inter alia* in the family *Gigartinaceae.* As used herein, the term "HOX" denotes an enzyme which is capable of oxidising the substrates selected from the group consisting of D-glucose, D-galactose, D-mannose, maltose, lactose and cellobiose.

HOX PRODUCTION

The gene encoding the HOX enzyme has been cloned from the seaweed *Chondrus crispus* (Stougaard and Hansen 1996, Hansen and Stougaard, 1997). The methylotrophic yeast *Hansenula polymorpha* (developed at Rhein Biotech, Dusseldorf/Germany as an expression system for heterologous proteins) has also been used to produce the HOX enzyme (the native protein was purified from seaweed (Poulsen and Høstrup, 1998)). WO 96/40935 and WO 98/13478 also disclose the cloning and expression in recombinant host organisms of a gene encoding a protein with HOX activity.

In one preferred embodiment the HOX enzyme comprises the sequence set out in SEQ ID No 22.

In one preferred embodiment the HOX enzyme comprises the sequence set out in SEQ ID No 22 or variants, homologues, derivatives or fragments thereof.

VARIANTS/HOMOLOGUES/DERIVATIVES (AMINO ACID SEQUENCE)

Preferred amino acid sequences are set out in SEQ ID No 22 or are sequences obtainable from the HOX enzyme mentioned herein but also include homologous sequences obtained from any source, for example related viral/ bacterial proteins, cellular homologues and synthetic peptides, as well as variants or derivatives thereof.

Thus, there is mentioned herein variants, homologues or derivatives of the amino acid sequences presented herein, as well as variants, homologues or derivatives of the nucleotide sequence coding for those amino acid sequences.

In the context herein, a homologous sequence is taken to include an amino acid sequence which is at least 75, 85 or 90% identical, preferably at least 95 or 98% identical at the amino acid level over at least, for example, the amino acid sequence as set out in SEQ ID No 22 of the sequence listing herein. In particular, homology should typically be considered with respect to those regions of the sequence known to be essential for enzyme activity rather than non-essential neighbouring sequences. These regions include but are not limited to the putative FAD binding domains in HOX such as $SGGH_{79}C$, $LGGH_{146}I$ and $LGGH_{320}A$. Although homology can also be considered in terms of similarity (i.e. amino acid residues having similar chemical properties/functions), in the context herein it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The terms "variant" or "derivative" in relation to the amino acid sequences mentioned herein includes any substitution of, variation of, modification of, replacement of, deletion of or addition of one (or more) amino acids from or

to the sequence providing the resultant amino acid sequence has an enzyme activity, preferably having at least the same enzyme activity as the amino acid sequence set out in SEQ ID No 22.

SEQ ID No 22 may be modified for use in the present invention. Typically, modifications are made that maintain the enzyme activity of the sequence. Amino acid substitutions may be made, for example from 1, 2 or 3 to 10 or 20 substitutions provided that the modified sequence retains the required enzyme activity. Amino acid substitutions may include the use of non-naturally occurring analogues.

SEQ ID No 22 for use in the present invention may also have deletions, insertions or substitutions of amino acid residues which produce a silent change and result in a functionally equivalent enzyme. Deliberate amino acid substitutions may be made on the basis of similarity in polarity, charge, solubility, hydrophobicity, hydrophilicity, and/or the amphipathic nature of the residues as long as the enzyme activity of the HOX enzyme is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

VARIANTS/HOMOLOGUES/DERIVATIVES (NUCLEOTIDE SEQUENCE)

It will be understood by a skilled person that numerous different nucleotide sequences can encode the same HOX enzyme as a result of the degeneracy of the genetic code. In addition, it is to be understood that skilled persons may, using routine techniques, make nucleotide substitutions that do not affect the HOX enzyme encoded by the nucleotide sequence for use in the invention to reflect the codon usage of any particular host organism in which the HOX enzyme is to be expressed.

The terms "variant", "homologue" or "derivative" in relation to the nucleotide sequence set out in SEQ ID No 22 includes any substitution of, variation of, modification of, replacement of, deletion of or addition of one (or more) nucleic acid from or to the sequence providing the resultant nucleotide sequence codes for a HOX enzyme having an enzyme activity, preferably having at least the same activity as the nucleotide sequence set out in SEQ ID No 22 of the sequence listings.

As indicated above, with respect to sequence homology, preferably there is at least 75%, more preferably at least 85%, more preferably at least 90% homology to the sequences shown in the sequence listing herein. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

There is also encompassed nucleotide sequences that are capable of hybridising selectively to the sequences presented herein, or any variant, fragment or derivative thereof, or to the complement of any of the above. Nucleotide sequences are preferably at least 15 nucleotides in length, more preferably at least 20, 30, 40 or 50 nucleotides in length.

HYBRIDISATION

The term "hybridization" as used herein shall include "the process by which a strand of nucleic acid joins with a complementary strand through base pairing" as well as the process of amplification as carried out in polymerase chain reaction (PCR) technologies.

Nucleotide sequences capable of selectively hybridising to the nucleotide sequences presented herein, or to their complement, will be generally at least 75%, preferably at least 85 or 90% and more preferably at least 95% or 98% homologous to the corresponding nucleotide sequences presented herein over a region of at least 20, preferably at

least 25 or 30, for instance at least 40, 60 or 100 or more contiguous nucleotides. Preferred nucleotide sequences will comprise regions homologous to the nucleotide sequence set out in SEQ ID No 22 preferably at least 80 or 90% and more preferably at least 95% homologous to the nucleotide sequence set out in SEQ ID No 22.

The term "selectively hybridizable" means that the nucleotide sequence used as a probe is used under conditions where a target nucleotide sequence is found to hybridize to the probe at a level significantly above background. The background hybridization may occur because of other nucleotide sequences present, for example, in the cDNA or genomic DNA library being screened. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e.g. with $^{32}P$.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5˚C (5˚C below the Tm of the probe); high stringency at about 5˚C to 10˚C below Tm; intermediate stringency at about 10˚C to 20˚C below Tm; and low stringency at about 20˚C to 25˚C below Tm. As will be understood by those of skill in the art, a maximum stringency hybridization can be used to identify or detect identical nucleotide sequences while an intermediate (or low) stringency hybridization can be used to identify or detect similar or related polynucleotide sequences.

In a preferred aspect, there is mentioned nucleotide sequences that can hybridise to the nucleotide sequence described herein under stringent conditions (e.g. 65˚C and 0.1xSSC {1xSSC = 0.15 M NaCl, 0.015 M $Na_3$ Citrate pH 7.0). Where the nucleotide sequence is double-stranded, both strands of the duplex, either individually or in combination, are encompassed. Where the nucleotide sequence is single-stranded, it is to be understood that the complementary sequence of that nucleotide sequence is also included within the description.

Nucleotide sequences which are not 100% homologous to the sequences mentioned herein but fall within the scope of the description can be obtained in a number of ways. Other variants of the sequences described herein may be obtained for example by probing DNA libraries made from a range of sources. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of the nucleotide sequence set out in SEQ I.D. No 22 under conditions of medium to high stringency. Similar considerations apply to obtaining species homologues and allelic variants of the amino acid and/or nucleotide sequences mentioned herein.

Variants and strain/species homologues may also be obtained using degenerate PCR which will use primers designed to target sequences within the variants and homologues encoding conserved amino acid sequences within the sequences mentioned herein. Conserved sequences can be predicted, for example, by aligning the amino acid sequences from several variants/homologues. Sequence alignments can be performed using computer software known in the art. For example the GCG Wisconsin PileUp program is widely used. The primers used in degenerate PCR will contain one or more degenerate positions and will be used at stringency conditions lower than those used for cloning sequences with single sequence primers against known sequences.

Alternatively, such nucleotide sequences may be obtained by site directed mutagenesis of characterised sequences, such as the nucleotide sequence set out in SEQ ID. No 22. This may be useful where for example silent codon changes are required to sequences to optimise codon preferences for a particular host cell in which the nucleotide sequences are being expressed. Other sequence changes may be desired in order to introduce restriction enzyme recognition sites, or to alter the enzyme activity of the HOX enzyme encoded by the nucleotide sequences.

The nucleotide sequences mentioned herein may be used to produce a primer, e.g. a PCR primer, a primer for an alternative amplification reaction, a probe e.g. labelled with a revealing label by conventional means using radioactive or non-radioactive labels, or the nucleotide sequences may be cloned into vectors. Such primers, probes and other fragments will be at least 15, preferably at least 20, for example at least 25, 30 or 40 nucleotides in length, and are also encompassed by the term nucleotide sequence as used herein.

The nucleotide sequences such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer nucleotide sequences will generally be produced using recombinant means, for example using a PCR (polymerase chain reaction) cloning techniques. This will involve making a pair of primers (e.g. of about 15 to 30 nucleotides) flanking a region of the targeting sequence which it is desired to clone, bringing the primers into contact with

mRNA or cDNA, performing a polymerase chain reaction (PCR) under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Due to the inherent degeneracy of the genetic code, other DNA sequences which encode substantially the same or a functionally equivalent amino acid sequence, may be used to clone and express the HOX enzyme. As will be understood by those of skill in the art, it may be advantageous to produce the HOX enzyme - encoding nucleotide sequences possessing non-naturally occurring codons. Codons preferred by a particular prokaryotic or eukaryotic host (Murray E et al (1989) Nuc Acids Res 17:477-508) can be selected, for example, to increase the rate of the HOX enzyme expression or to produce recombinant RNA transcripts having desirable properties, such as a longer half-life, than transcripts produced from naturally occurring sequence.

SCREENS

The method described herein may be used for screening for elevated levels of the intracellular POI in mutated host cell organisms. The cells employed in such a screen may be affixed to a solid support or on a solid substrate, such as plastic pins or some other surface. The cells may be contacted with the membrane extracting composition mentioned herein and the level of the released POI may be measured using methods known in the art.

HIGH THROUGH PUT SCREENS (HTS)

The method described herein may be used in high through-put screening (HTS) systems, where target cells are grown and screened in microtiter plates (10000 mutants per day) by robot systems. By way of example, when making new recombinant production strains, it is usually necessary to carry out one or several rounds of traditional mutagenesis in order to increase productivity. This is most efficiently done using HTS of the mutated cells.

The method described herein is highly advantageous because it allows for high through put screening (HTS) for increased levels of intracellular POIs. Up until now, these systems were only able to screen for higher levels of secreted POIs.

INTRODUCTION TO THE EXAMPLES SECTION AND THE FIGURES

The present invention will now be described only by way of example in which reference is made to the following Figures.

Figure 1 provides a genetic construct;

Figure 2A provides genetic constructs;

Figure 2B provides a photographic representation;

Figures 3A and 3B provide photographic representations;

Figure 4 provides a graph;

Figure 5 provides a sequence listing;

Figure 6 provides a sequence listing;

Figures 7A-7D provide a photographic representation;

Figure 8 provides a graph;

Figure 9 provides a graph;

Figures 10A-10B provide a photographic representation;

Figures 11A-11B provide a graph;

Figures 12A-12B provide a photographic representation;

Figures 13A-13B provide a photographic representation; and

Figures 14A-14B provide a photographic representation.

In slightly more detail:

Figure 1 provides a physical map of the expression vector for HOX production in *Hansenula polymorpha.* EcoRI/NotI blunt fragments harbouring the coding region of the synthetic HOX gene fused to optional signal sequences were cloned into the multiple cloning site of a standard *Hansenula* expression vector. The expression vectors contain the promoter of formate dehydrogenase (FMD) gene and the terminator (MOX-T) of the methanol oxidase gene separated by the multiple cloning site for fragment insertion, ori and bla (ampR) for propagation and selection in *E. coli,* the ARS (HARS) sequence for replication in *H. polymorpha,* the URA3 gene for selection.

Figure 2A shows a diagram of the 1.4kb genuine FMD gene (upper scheme) and the FMD promoter with the cloned heterologous DNA (lower scheme). The restriction sites are Asp718, NcoI.

Figure 2B shows the gene copies of the integrated HOX gene. Lanes 1-12 show different recombinant isolates and their corresponding DNA dilution. Lane 13 shows an untransformed host strain and Lane 14 shows a size marker (M).

Figures 3A & 3B provide an SDS-PAGE analysis of HOX expression. Figure 3A provides an SDS-PAGE analysis of culture filtrate from glycerol fermentation of the mutagenized strain DK8-27KanII3-mut25. Lane 1 shows a marker protein, lane 2 shows a HOX standard (0.03U/ml; 18ul), lane 3 shows a supernatant from probe 3 (18ul), lane 4 shows a supernatant from probe 4 (18ul), lane 5 shows a supernatant from probe 5 (18ul), lane 6 shows a supernatant from probe 6 (18ul), lane 7 shows a supernatant from probe 7 (18ul), lane 8 shows a supernatant from probe 8 (18ul), lane 9 shows a supernatant from probe 9 (18ul) and lane 10 shows a supernatant from probe 10 (18ul).

Figure 3B provides a western blot analysis of recombinant strains expressing HOX. The samples applied in the lanes are the same as for Figure 3A. The membrane was probed with a polyclonal HOX antibody.

Figure 4 shows the growth and productivity of a 10 liter fermentation culture of a secreting strain DK8-27KanII3-mut25. The fermentation was performed at 25˚C and pH 5.0 with glycerol and $pO_2$ control.

Figure 5 provides the individual oligonucleotides used to synthesize the HOX gene with codon optimization.

Figure 6 provides a nucleotide squence of the synthetic HOX gene and the corresponding amino acid sequence.

Figures 7A-7D show the localisation of the HOX enzyme in *H. polymorpha* as determined by immunofluorescence. The superimposition of the location of the HOX enzyme (green signal) with the nuclear location (blue signal) is shown. See A) RB11 strain without HOX gene. B) DK8-27. C) DK8-27 mut25. D) DK2II-I.

Figure 8 provides a graph showing HOX activity as a function of number of cycles through a cell homogenizer.

Figure 9 provides a graph showing *Hansenula polymorpha* cells extracted with different concentration of CTAB and Triton X-100.

Figure 10A shows an SDS PAGE analysis of HOX enzyme levels in the cell supernatant (lanes 7-10) and pellet (lanes 2-5) after CTAB treatment. The HOX enzyme was released from the pellets by mechanical extraction. The samples were analysed on 4-12% NuPAGE gels from MES, Novex and 10 μl samples were loaded in each lane in the following order: Lane 2-5: residual HOX in the cell pellet; Lane 7-10: released HOX in the supernatant; Lane 1 and 6: Novex See Blue standard; Lane 2: control, lane 3: 0.1% CTAB; lane 4: 0.2% CTAB; lane 5: 0.4% CTAB; lane 7: control; lane 8: 0.1% CTAB; lane 9: 0.2% CTAB and lane 10: 0.4% CTAB.

Figure 10B shows a Western Blot analysis of HOX enzyme levels in the cell supernatant (lanes 7-10) and pellet (lanes 2-5) after CTAB treatment. The HOX enzyme was released from the pellets by mechanical extraction. The samples were analysed on 4-12% NuPAGE gels from MES, Novex and 5 μl samples were loaded in each lane in the following order: Lane 2-5: residual HOX in the cell pellet; Lane 7-10: released HOX in the supernatant; Lane 1

and 6: Novex See Blue standard, Lane 2: control, lane 3: 0.1% CTAB; lane 4: 0.2% CTAB; lane 5: 0.4% CTAB; lane 7: control, lane 8: 0.1% CTAB; lane 9: 0.2% CTAB and lane 10: 0.4% CTAB.

Figure 11A shows the elution profile for CTAB extracted HOX.

Figure 11B shows the elution profile for mechanically extracted HOX.

EXAMPLES

**Materials and Methods**

**Chemicals**

All chemicals used were of analytical reagent grade. Lecithin (3-*sn*-phosphatidylcholine) was commercially available as Stempur PM from Stem (Germany). Pronase E (a proprietary name for a mixture of various exo- and endopeptidases, obtained from *Streptomyces griseus,* that is able to hydrolyse virtually any protein almost completely to free amino acids). Lysolecithin (lysophatidylcholine), D-glucose, o-dianisidine, peroxidase (P-8125), capric acid (decanoic acid), saponin (any member of a large group of glycosides, widely distributed in plants, that are powerful surfactants) and CTAB (cetyltrimethylammonium bromide also known as hexadecyltrimethylammonium bromide) (H-5882) were all from Sigma Chemical Co., USA. Methanol (HPLC) was from Lab-Scan Ltd. Hydrogen peroxide and Triton X-100 (a proprietary name for polyethoxylated octylphenol) were from Merck, Germany. Emulsifier YN also commercially known as Palsgaard 4445 was from Palsgaard, Denmark. The quaternary ammonium compounds such as LTAB (lauroyltrimethylammonium bromide), Cetrimide-40 (also known as cetrimidum which is a detergent disinfectant consisting of a mixture of alkylammonium bromides, principally CTAB), CTAB (cetyltrimethylammonium bromide), STAB (stearoyl trimethyl ammonium bromide), MTAC (myristyl trimethyl ammonium chloride), CTAC (Cetyl Trimethyl Ammonium Chloride), STAC (stearoyl trimethyl ammonium chloride) were all from FeF, Denmark. Rodalon comprises about 9.5% (95 g/l) alkyldimethylbenzylammonium chloride ($C_{12}H_{25}N(CH_3)_2C_7H_7Cl$) was obtained from Superfos Biosector, 2950 Vedbaek, Denmark. Alkyldimethylbenzylammonium chloride is also known as benzalkoniumchloride. The emulsifier Sodium Lauroyl Lactylate (SLL) was from Danisco Cultor, Grindsted, Denmark.

**Yeast fermentation**

The cultivation of yeast was performed in a 6 L or a 100 L fermentor according to Rhein Biotech fermentation manual for 10 L scale.

**Example 1**

**Assembly of a synthetic, codon optimized HOX gene**

*Gene Design*

The nucleotide sequence of the native HOX gene was altered resulting in a synthetic gene. The synthetic HOX gene (Figure 6) was designed so that the codon usage was precisely matched to the known codon preferences of biotechnologically relevant yeasts such as *Pichia sp., Hansenula sp., Kluyveromyces, Yarrowinia, S. Pombe* in order to facilitate high level production in these organisms. The gene was divided into three separately assembled and/or cloned fragments. The sub-assemblies, designated as 5' proximal half were comprised of the following oligonucleotides as set out in Figure 5 as complementary pairs: HOX1a/HOX2b, HOX3a/HOX4b, HOX5a/HOX6b, HOX7a/HOX8b, HOX9a/HOX10b); 3'distal half A using primers 1-6 and 3'distal half B using primers 6-10.

*5' proximal synthetic HOX gene*

The 5'proximal half of the synthetic HOX gene was synthesized using ten oligonucleotides HOX1A to HOX10B. The oligonucleotides having lengths ranging from 100-120 base pairs were used as primers (concentration = 0.1 $\mu$M each) in a hot start PCR reaction of 100 $\mu$L (using the thermostable DNA polymerase Pwo (Boehringer). Hot start was performed by heating the mixture of oligonucleotides, buffer, $MgSO_4$ to 90˚C before dNTP (250$\mu$M) and *Pwo* polymerase (2.5 units) was added. 40 cycles of PCR using the PCR profile: 94˚C for 30 seconds, 57˚C for 1 minute and 72˚C for 1 minute. A 10 minute elongation step at 72˚C was included at the end of the 40 cycles. Analysis of the products from this PCR in agarose gel electrophoresis showed a smear of DNA bands ranging in size from 100 to 850 base pairs. The first

PCR was reamplified using 2ul from the above reaction as template and the flanking primers (1 μM each) HOX1A and HOX 10B. The reaction contained 200 μM dNTP, 2.5 mM $MgCl_2$ and 2 units of AmpliTaq® (Perkin-Elmer Cetus). The PCR conditions were: 94˚C for 2 minutes, then 30 cycles of PCR with the profile 94˚C for 30 seconds, 60˚C for 1 minute and 72˚C for 45 seconds. A 10 minutes elongation step at 72˚C was included at the end of the above reaction. Analysis of the second PCR product by agarose gel electrophoresis showed the presence of a 850 bp DNA band which was subsequently purified from the gel and cloned into the vector pCR® (Invitrogen).

*3' distal synthetic HOX gene*

Ten primers of lengths ranging from 90-126 base pairs were designed to synthesize the distal part of the HOX gene. The primers contained overlapping (complementary) regions of 16-21 base pairs with a calculated melting temperature of approximately 60˚C. The distal part of the HOX gene was synthesized as two fragments (A & B), each with a size of 530 base pairs. Two PCR reactions were performed using 6 primers at a time. The PCR reaction 1 contained primers 1-6 and PCR reaction 2 contained primers 5-10. The PCR amplification reactions were performed using 0.1 μM of each of the primers, 250 μM each dNTP, 2mM $MgSO_4$ and 2.5 units of Pfu DNA polymerase from *Pyrococcus furiosus* (Strategene) in a reaction volume of 100 μl. The cycling parameters for the 2 PCR reactions using Pfu DNA polymerase included a 1 minute denaturation at 95˚C followed by 30 cycles of PCR: 94˚C for 1 minute, 55˚C for 1 minute and 72˚C for 1 minute. This was followed by an elongation step at 72 ˚C for 3 minutes. Analysis of the PCR products from the two PCR reactions by agarose gel electrophoresis showed in both cases, the synthesis of one specific DNA band of the correct size of approximately 530 bps in length. The PCR products were cloned in pCR ® -Blunt vector (Invitrogen). The cloned partial synthetic HOX genes were sequenced using primers flanking the multiple cloning sites (M13 reverse primer and T7 promoter primer). The sequencing results verified that the synthesized partial genes contained the correct sequence.

*Assembly of the final codon-optimized HOX gene*

The three parts of the synthetic HOX gene were combined by ligation of the gel purified DNA fragments comprising of the Ncol/PvuII 5' proximal HOX, the 3' distal PvuII/SpeI HOX fragment A and fragment B cut with SpeI/NotI. The complete, codon optimized synthetic HOX gene (Figure 6) was assembled into the *Hansenula* expression vector, which was developed to mediate the expression and secretion of foreign proteins from *Hansenula.* The expression vector is based upon the formate dehydrogenase promoter (FMD), the MOX terminator, with and without a yeast secretion signal.

**Results 1**

**Expression of the recombinant HOX in *H. polymorpha***

Table 1 shows the various HOX/secretion fusion constructs which were inserted as Eco RI/Not I blunt fragments into the multiple cloning site of the *H. polymorpha* expression/integration vector. The different signal sequences were derived from the glucoamylase gene from *Schwanniomyces occidentalis,* α-factor mating type gene from *Saccharomyces cerevisiae* and the TAKA-amylase from *Aspergillus oryzae.* A NcoI/NotI HOX construct without a signal sequence was also cloned into the vector.

| Name of Clone | signal sequence | HOX | Fusion junction |
|---|---|---|---|
| 1. DK 1 | glucoamylase | wildtype synthetic | SAIQA MATLP |
| 2. DK 2 | glucoamylase | wildtype synthetic | SAIQA ATLP |
| 3. DK 3 | α-factor | wildtype synthetic | KREAEA MATLP |
| 4. DK 4 | α-factor | wildtype synthetic | KREAEA ATLP |
| 5. DK 5 | α-factor | mutant synthetic | KREAEA MATLP |
| 6. DK 6 | α-factor | mutant synthetic | KR MATLP |
| 7. DK 7 | TAKA amylase | mutant synthetic | APALA MATLP |
| 8. DK 8 | No signal sequence | wild type synthetic | none - MATLP |

The term mutant synthetic relates to a putative KEX 2 protease cleavage site $R_{331}$-$K_{332}$ to $R_{331}$-$P_{332}$.

**Example 2**

**Transformation and passaging**

The different HOX expression plasmids were used to transform the uracil auxotrophic *H. polymorpha* strain RB11 to uridine prototrophy. The HOX transformants harbouring the different expression plasmids were cultivated under selective conditions for 30 generations to amplify the plasmid DNA and allow integration into the genome. The transformants were grown on complete non-selective medium for 20 generations. In addition to the selection, PCR and southern analysis were used to characterize the transformants.

**Copy number determination of the integrated heterologous DNA**

The genomic DNA of the untransformed host strain and the various recombinant isolates of a particular HOX construct were digested with the restriction enzymes, Asp718/NcoI. The restricted DNA was separated on 0.8% agarose gels, transferred to membrane (nitrocellulose) and hybridized to a $^{32}P$-labelled fragment of the cloned FMD promoter. The hybridization pattern reveals two signals, one for the genuine single copy 1.4kb FMD gene and one originating from the slightly smaller heterologous fusion. A series of dilutions enabled the estimation of the signal intensity of the integrated DNA compared to the intrinsic single copy control.

**Results 2**

**Screening for HOX expression**

Transformants were grown in 3 mL tube cultures and cultivated under derepressing conditions by supplementing the medium with 1% glycerol. HOX expression was analysed by SDS-PAGE analysis of cultures from glycerol fermentation. Western blot analysis using a polyclonal HOX antibody was used to detect the presence of HOX protein.

**Table 2.** Characteristics of selected transformants expressing HOX.

| Transformant | Copy Number | N-terminus | Localization of expression | HOX Activity |
|---|---|---|---|---|
| DK1-49 | ≈10 | unprocessed signal peptide | soluble & insoluble fractions | none |
| DK 2II-1 | ≈ 20 | unprocessed signal peptide | soluble & insoluble fractions | none |
| DK3II-4 | ≈ 20 | unprocessed signal peptide | soluble & insoluble fractions | none |
| DK4-39 | ≈ 10 | unprocessed signal peptide | soluble & insoluble fractions | none |
| DK5-13 | ≈ 30-40 | unprocessed signal peptide | soluble & insoluble fractions | none |
| D6-16 | ≈ 10 | unprocessed signal peptide | soluble & insoluble fractions | none |
| DK7-1 | ≈ 10 | unprocessed signal peptide | soluble & insoluble fractions | none |
| DK8-1 | ≈ 2-3 | same as mature | soluble & insoluble fractions | active |
| DK8-27 | ≈ 20 | same as mature | soluble & insoluble fractions | active |
| DK8-27* Kan II3-mut25 | ≈ 20 | same as mature | extracellular intracellular | active active |

(continued)

| Transformant | Copy Number | N-terminus | Localization of expression | HOX Activity |
|---|---|---|---|---|
| DK8-27Kan* IV2-mut301 | ≈ 20 | same as mature | extracellular intracellular | active active |
| *The strain DK8-27 was subjected to chemical mutagenesis (NTG-nitrosoguanidine). | | | | |

## Example 3

### Localisation of recombinant HOX in *H. polymorpha*

For immunofluorescence microscopy of recombinant *H. polymorpha,* cells were precultured in Yeast Nitrogen Base (YNB) + glucose to a density of $10^8$ cells/ml. To induce expression, 3 x $10^8$ cells were shifted to 100 mL shake flask cultures supplemented with YNB ± 1% glycerol. After 1, 2 or 3 days of growth under derepressing condition 5x$10^8$ cells were fixed by a combined para-formaldehyde (4%) and glutaraldehyde (0.2%) treatment (Hagen and Hyam, 1988). After three washes with 1 mL of PEM (100 mM Pipes, 1 mM EGTA, 1 mM $MgSO_4$, pH 6.9), the cell walls were partially removed in PEMS (PEM + 1 M sorbitol) supplemented with 0.5 mg/mL Zymolyase-100T. After approximately 60 minutes of digestion, cells were shifted to PEMS + 1% Triton X-100, incubated 30 seconds and washed three times with 0.5 mL PEM. To quench unreacted glutaraldehyde cells were resuspended in PEM + 1 mg/mL sodium borohydride. Immediately after this, cells were washed twice in PEM, resuspended in PEMBAL (PEM + 1% BSA (globulin free), 1 mM lysine hydrochloride, 0.1% $NaN_3$), and incubated on a rotating wheel for 30 minutes. 25% of the cell suspension, equalling $10^8$ cells, was supplemented with 10 μg/ml of affinity purified polyclonal anti-HOX antibodies and incubated overnight at room temperature. After three washes in 0.5 mL PEMBAL, cells were suspended in PEMBAL, and incubated 5-20 hours in the dark with 0.5% FITC-conjugated goat anti-rabbit antibodies (Sigma). After wash in PEMBAL, the cells were washed once in PBS, once in PBS + 0.2 μg/mL diamidinophenylindole (DAPI) and finally resuspended in PBS + 0.1% $NaN_3$. For microscopic observation, small samples of cell suspensions were dried onto poly-L-lysine coated coverslips and inverted into drops of 100% glycerol containing 1 mg/mL para-phenylene diamine. Cells were examined with a Zeiss microscope equipped for indirect immunofluorescence at 1.000 X and images were captured by a CCD camera (Micro-MAX Kodak) and processed using MetaMorph software.

### Results 3

Immunofluorescence microscopy of the DK8-27 transformant revealed that the recombinant HOX protein primarily localises to the periphery of the cell as aggregates (Figure 7b). Combined with the biochemical data, these results indicate that HOX to some extent may be a membrane associated protein (as opposed to a substantially membrane bound protein). It is most likely that HOX localises to the plasma membrane in *H. polymorpha.* Also, in the DK8-27 mut25 strain, which is derived from DK8-27, HOX is associated with the plasma membrane (Figure 7c). The protein, however, does not accumulate in aggregates but is more uniformly distributed. When fused to various leader peptides HOX accumulates in huge intracellular aggregates (Figure 7d)

## Example 4

### Extraction of HOX from recombinant *Hansenula* cells by means of different detergents and proteases

The experiment was carried out by using 5.0 mL cell suspension (cells + supernatant) in a 15 mL centrifuge tube (HOX9926-7, 317 g cells/L wet weight, 0.3 U/mL extracellular HOX activity). Cells were separated by centrifugation at 4000 g for 10 min. For permeabilisation experiments, the supernatant was then supplemented with either, CTAB, CTAB+Pronase E, Pronase E, Tween 20 (a proprietary name for polyoxyethylene sorbitan monolaurate) and Tween 80 (a proprietary name for sorbitan monooleate). The cells were then resuspended in 4.0 mL supernatant and incubated for 23 hours at 25˚C (500 rpm). In order to examine the effect of time with CTAB, the cells in one of the tubes were only incubated for 7 min at 25˚C in 4 mL 0.4% CTAB. The cells were then separated by centrifugation. The cells were then re-suspended again in the original supernatant without CTAB added and then incubated for 23 hours as above. After incubation, the extracellular HOX in the cell-free extracts was measured by the HOX assay.

**Assay method for determination of HOX activity (HOX assay)**

HOX activity was estimated by the assay of Sullivan and Ikawa (1973). The assay was scaled down to be run in microtiter plates.

**Principle**

The HOX assay is based on the measurement of hydrogen peroxide generated in the oxidation of glucose. The hydrogen peroxide oxidizes o-dianisidine in presence of peroxidase (POD) to form a dye.

$$\beta\text{-D-glucose} + H_2O + O_2 \xrightarrow{\text{HOX}} \text{D-glucono-delta-lactone} + H_2O_2$$

$$H_2O_2 + o\text{-dianisidine}_{red.} \xrightarrow{\text{POD}} 2\,H_2O + o\text{-dianisidine}_{ox.}$$

**Reagents**

1. 100 mM phosphate buffer, pH 6.3
2. 100 mM D-glucose in 100 mM phosphate buffer, pH 6.3
3. *o*-Dianisidine, 3.0 mg/mL in distilled water
4. Peroxidase, 0.10 mg/mL in 100 mM phosphate buffer, pH 6.3

**Assay**

120 μl reagent 1
150 μl reagent 2
10 μl reagent 3
10 μl reagent 4
and 10 μl enzyme solution (in proper dilution)

The assay is performed in a microtiter plate. The reaction is initiated by the addition of enzyme solution. The mixture is incubated at 25˚C for 10 min with shaking. The blank run contains all the components with water instead of enzyme solution. The formation of the dye is measured in a microtiter plate reader at 405 nm. The linearity of the reaction is checked by using a kinetics programme on the microplate reader.

**Hydrogen peroxide standard curve**

A hydrogen peroxide standard curve is constructed by using varying concentrations of fresh $H_2O_2$.

One unit of enzyme activity is defined as the amount of enzyme which produces 1 μmol of $H_2O_2$ per min at 25˚C.

**Results 4**

The data presented in Table 3 shows that CTAB is very efficient in extracting HOX. CTAB is also much more efficient than Tween 20 and Tween 80. There is no significant benefit of adding a protease. Very interestingly CTAB exerts its positive effect even when used only for a 7 min preincubation, this indicates that CTAB very quickly binds to and permeabilizes the *Hansenula* cell wall. This is supported by analysis of the cell free supernatant for CTAB (see below) which shows that only 50-100 ppm out of 4000 ppm CTAB added is present in the cell free supernatant.

A comparison of the sediment in the centrifuge tubes for each test agent also indicates that the packed cell volume of the CTAB treated cells is smaller than the volume of the control cells or cells treated with detergents other than CTAB. This shrinkage of the cells indicates that the cells have indeed been permeabilized and emptied for some of their soluble content.

Table 3

Effect of detergent, detergent in combination with protease and pre-incubation on the extraction of intracellular HOX

| Test | HOX activity % |
|---|---|
| Control | 100 |
| 0.4% CTAB | 4600 |
| 0.4% CTAB + Pronase E (400 PU) | 5000 |
| 0.4% CTAB + Pronase E (800 PU) | 4800 |
| Pronase E (400 PU) | 120 |
| 0.4%Tween 20 | 140 |
| 0.4%Tween 80 | 140 |
| Pre-incubation in 0.4% CTAB for 7 min | 5100 |

**Example 5**

**Extraction of HOX using CTAB and benzalkonium chloride (BAC)**

The experiment was carried out by using a 5.0 mL cell suspension (cells + supernatant) in a 15 mL centrifuge tube (HOX9959, Mut 45). The cell suspension was then supplemented either with CTAB (from a 10% CTAB stock solution) or benzalkonium chloride (Rodalon, 9.5% benzalkonium chloride) and incubated for 22 hours at 25˚C (200 rpm). After incubation, extracellular HOX (cells were removed by 10 min centrifugation at 4000 g) was measured by HOX assay.

**Results 5**

The data presented in Table 4 indicate that benzalkonium chloride (BAC) is very effective in releasing the HOX enzyme from the cells.

Table 4

Effect of CTAB and Benzalkonium chloride (BAC) on HOX release from cells

| Test | HOX activity, % |
|---|---|
| Control | 100 |
| 0.4% CTAB | 1300 |
| 0.08% BAC | 2500 |
| 0.17% BAC | 2300 |
| 0.50% BAC | 2300 |
| 0.70% BAC | 2100 |
| 0.83% BAC | 1700 |
| 1.00% BAC | 1600 |

**Example 6**

**Extraction of HOX by CTAB combined with salts and at different temperatures**

In order to examine the mechanism of the CTAB effect, CTAB was combined with chaotrophic and nonchaotrophic salts.Five mL cell suspension (cells + supernatant) was added to a 15 mL centrifuge tube (HOX9926-7, 317 g cells/litre wet weight, 0.3 U/mL extracellular HOX activity). Cells were separated by centrifugation at 4000 g for 10 min. The supernatant was then supplemented with either CTAB, CTAB+NaCl, CTAB+urea, CTAB+ammonium sulphate, or

the non-ionic detergent, octyl-glucoside. The cells were then re-suspended in 4.0 mL supernatant and incubated for 26 hours at 25˚C (500 rpm). In this experiment, the effect of shaking and temperature was also investigated. After incubation, the cell-free extract was used to estimate HOX activity using the HOX assay as outlined in Example 4.

**Results 6**

The results are shown in Table 5. It is clear that shaking is not necessary in order to have extraction of HOX in the presence of CTAB. There is a clear temperature effect, meaning that extraction at 4˚C results in only half the activity extracted at 25˚C. The addition of sodium chloride and ammonium sulphate both decrease the effect of the CTAB treatment, which may indicate that the ionic nature of CTAB is important. The addition of urea had a less drastic effect but still reduced the amount of extracted HOX to approximately half of the amount extracted with 0.4% CTAB. Although urea is non-ionic, it may interfere with hydrophobic interaction Urea has been reported in the prior art as a means of permeabilizing *Pichia* cells for extraction of lipophilic proteins (Craig 1987). The non-ionic detergent octyl glucoside has no significant extracting effect.

Table 5

Effect of detergent, detergent in combination with salt, shaking, and temperature on the extraction of intracellular HOX.

| Test | HOX activity, % |
|---|---|
| Control | 100 |
| 0.4% CTAB | 6700 |
| 0.4% CTAB, without shaking | 7700 |
| 0.4% CTAB, without shaking, at 4 ˚C | 2800 |
| 0.4% CTAB + 1.0 M NaCl | 1900 |
| 0.4% CTAB + 1.0 M urea | 3600 |
| 0.4% CTAB + 1.0 M ammonium sulphate | 2300 |
| 0.2% octyl-glycoside | 130 |
| 0.4% octyl-glycoside | 190 |

**Example 7**

**Determination of CTAB and LTAB by LC-ESI-MS in cell extracts containing HOX**

The samples of extracted HOX from Example 5 were analyzed for their content of CTAB by means of LC-ESI-MS on a Hewlett-Packard 1100 HPLC-MS system consisting of the following units:

a) Binary gradient pump, HP 1100
b) Autosampler, HP 1100,
c) Thermostated Column Compartment, HP 1100
d) Mass Selective Detector, HP 1100
e) Chromatographic data system, HP ChemStation, Version 6.01

The system was equipped with a Zorbax Eclipse® XDB-C8, 5 μM, 150 x 4.6 mM id. (Hewlett-Packard) column. Column temperature was 25˚C.

The chromatographic conditions were a mobile phase consisting of two solvents. Solvent A: 1 mM $NH_4OAc$/Water, solvent B: I mM $NH_4OAc$/Methanol. The column was run with isocratic conditions (that is, using conditions where the composition of the eluant is maintained constantly during the chromatographic period): 5% A + 95% B, with a solvent flow rate of 0.80 mL/min and an injection volume of 10 μL. The samples were injected directly.

The mass spectrometric conditions were with the following spray chamber settings:

Ionisation mode: Electrospray in positive mode
Drying gas ($N_2$) temperature: 350 ˚C
Drying gas flow rate: 6.0 l/min
Nebuliser pressure: 60 psi
Capillary voltage: - 4000 Volts
Fragmentor voltage: 100 Volts

The detector settings were the following: SIM parameters: m/z 284.1 (hexadecyltrimethylammonium cation). A stock solution containing 500 μg CTAB/mL water (concentration index 1000) was diluted with water to obtain standard solutions with the following concentration indices: 300 - 100 - 30 - 10. To the samples was added 0.4% CTAB which would give 4000 μg/mL if all the CTAB was present in the extract.

The analysis method for the quaternary ammonium compounds was optimised by using a different column, and by using different mobile phase. Two 90 L scale fermentations (Vest0002b with a biomass concentration of 314 g/L wet cells and Vest0003b with a biomass concentration of 332 g/L wet cells) were added LTAB to a concentration of 0.20 % (w/v), and HOX was extracted for 24 h. A sample from each fermentation was centrifuged at 10000 g for 10 minutes, and the resulting supernatants were withdrawn for LTAB analysis. The following method was used to quantify the LTAB content in the supernatants by means of LC-ESI-MS on a Hewlett-Packard 1100 HPLC-MS system consisting of the following units:

a) Binary gradient pump, HP 1100
b) Autosampler, HP 1100,
c) Thermostated Column Compartment, HP 1100
d) Mass Selective Detector, HP 1100
e) Chromatographic data system, HP ChemStation, Version 6.01

The system was equipped with a PLRP-S, 100Å, 5 μm, 250 x 4.6 mM id. (Polymer Laboratories) column. Column temperature was 25˚C.

The chromatographic conditions were a mobile phase consisting of 0.1% heptafluorobutyric acid in methanol. The column was run with a solvent flow rate of 1.00 mL/min and an injection volume of 5 μL. The samples were diluted 25 fold with methanol and filtered through Gelman GHP Acrodisc 13 mM Minispike 0.45 μM before injection.

The mass spectrometric conditions were with the following spray chamber settings:

Ionisation mode: Electrospray in positive mode
Drying gas ($N_2$) temperature: 350˚C
Drying gas flow rate: 13.0 L/min
Nebuliser pressure: 60 psi
Capillary voltage: - 4000 Volts
Fragmentor voltage: 150 Volts

The detector settings were the following: SIM parameters: m/z 228.1 (lauroyltrimethylammonium cation). A stock solution containing 250 μg LTAB/mL methanol (concentration index 1000) was diluted with methanol to obtain standard solutions with the following concentration indices: 400 - 200 - 120 - 80 - 36 - 10.8 - 5.4 - 2.16 - 0.864.

**Results 7**

It is clear from Table 6 that the level of CTAB in cell extracts containing the HOX enzyme is much lower than the amount added to the cells. This is explained by the binding and therefore immobilization of the CTAB to the yeast cell walls. This means that the resulting HOX enzyme only contains a very low level of CTAB.

Table 6

| Content of CTAB in the extracted HOX supernatants from Example 6 | |
|---|---|
| Test | [1]CTAB concentration, μg/mL |
| Control no CTAB added | 21 |
| 0.4% CTAB | 115 |
| 0.4% CTAB, without shaking | 52 |
| 0.4% CTAB, without shaking, at 4˚C | 35 |
| 0.4% CTAB + 1.0 M NaCl | 212 |
| 0.4% CTAB + 1.0 M urea | 235 |
| 0.4% CTAB + 1.0 M ammonium sulphate | 246 |
| [1]Anatysed by the first method | |

The results obtained on LTAB in the supernatant (see Table 6a) show that only about 27% of the added LTAB

is found in the cell free fraction. This result shows the same tendency as the results with CTAB in Table 6.

Table 6a

Content of LTAB in the supernatants extracted from fermentation Vest0002b and Vest0003b from Example 6

| Fermentation | LTAB added [μg/mL] | [1]LTAB. in cell free extract [μg/mL] |
|---|---|---|
| Vest0002b | 2000 | 538 |
| Vest0003b | 2000 | 550 |

[1]Analysed by the optimised method

**Example 8**

**Effect of temperature on time end efficiency of HOX extraction by CTAB**

The effect of temperature on time end efficiency of HOX extraction by CTAB was examined on a *Hansenula* sample: Mut 45, HOX9949, 282 g/L, 2.6 U/mL.

To 5 mL of ferment (cells + supernatant) in a centrifuge tube, either 0.2% or 0.4% CTAB (from a 10% CTAB solution) was added. The tubes were incubated at 25, 30, 35 and 40˚C, respectively (200 rpm). At the indicated times samples were taken and after centrifugation for 5 min at 10000 g, the supernatant was assayed for HOX activity. The results are shown in Table 7.

Table 7

Time course of HOX extraction from *H. polymorpha* at different temperatures

| Extraction conditions | Extracted HOX [U/mL] | | | | |
|---|---|---|---|---|---|
| | 4h | 8h | 24 h | 31 h | 48 h |
| 25˚C, 0.2% CTAB | 5.1 | 7.5 | 31 | 36 | 44 |
| 25˚C, 0.4% CTAB | 5.9 | 9.2 | 25 | 29 | 37 |
| 30˚C, 0.2% CTAB | 6.8 | 15 | 38 | 45 | 44 |
| 30˚C, 0.4% CTAB | 7.4 | 15 | 36 | 40 | 42 |
| 35˚C, 0.2% CTAB | 6.4 | 16 | 36 | 44 | 41 |
| 35˚C, 0.4% CTAB | 8.2 | 15 | 33 | 37 | 23 |
| 40˚C, 0.2% CTAB | 16 | 27 | 44 | 43 | 32 |
| 40˚C, 0.4% CTAB | 17 | 28 | 56 | 59 | 40 |

**Results 8**

It is clear that CTAB extraction is dependent on the temperature and that a faster extraction can be achieved by using a higher temperature. This is, however a parameter which has to be balanced with the stability of the extracted protein. In this experiment no significant difference seems to exist between using 0.2% or 0.4% CTAB. However, this depends on the cell concentration in the specific experiment.

**Example 9**

**HOX extraction with different quaternary ammonium compounds**

Several quaternary ammonium compounds were tested with respect to extraction of the intracellular HOX enzyme from *Hansenula polymorpha.* A sample of fermentation broth was withdrawn from a 6 L scale fermentation where the biomass concentration was approximately 340 g wet weight per L. One mL of a 4% (w/v) solution of each of the quaternary ammonium compounds listed in Table 8 was added to 9 mL of fermentation broth in plastic tubes. After 24 h of incubation at 25˚C at 200 RPM the tubes were centrifuged 10 min. at 12000 g. The supernatants were analysed for HOX activity using the HOX assay as previously described.

The time course of HOX extraction was studied with CTAB, LTAB and CTAC. A fermentation sample containing 280 g wet weight of *Hansenula polymorpha* per L was withdrawn from the fermentor. A 4% (w/v) solution of CTAB, LTAB and CTAC was added to a final concentration of 0.2 or 0.4% (w/v) to plastic tubes containing 9 mL of fermentation

broth. After 0, 7, 17, 24, and 48 h of incubation at 25˚C at 200 RPM the tubes were centrifuged 10 min. at 12000 xg. The supernatants were analysed for HOX activity using the HOX assay as previously described.

The extracting effect of LTAB was tested on the *Pichia pastoris* strain #349 that produces HOX intracellularly. A sample of fermentation broth was withdrawn from a 6 L scale fermentation where the biomass concentration was approximately 232 g wet weight per L. Nine mL of fermentation broth was added to plastic tubes together with 0 (control) or 180 μL of a 10% (w/v) solution of LTAB. After 24 h of incubation at 30˚C at 20 RPM the tubes were centrifuged 5 min. at 9000 g. The supernatants were analysed for HOX activity using the HOX assay as previously described.

**Results 9**

HOX could be extracted with all the tested quaternary ammonium compounds (see Table 8) when added to a fermentation sample in a final concentration of 0.4% (w/v). After 24 h of incubation at 25˚C, LTAB was superior to the other tested compounds with respect to extraction of HOX. The amount of HOX extracted seemed to decrease with increasing chain length of the quaternary ammonium compound.

The time course of HOX extraction with CTAB, LTAB or CTAC is shown in Table 9. It is clear that both incubation time and the concentration of the extraction reagent influences the amount of HOX activity extracted. LTAB is found to be the best extraction reagent at all analysed incubation times which is consistent with the results shown in Table 8. The extraction of HOX with LTAB seems to proceed at a slower pace at a concentration of 0.2% (w/v) LTAB, than at a concentration of 0.4% (w/v) LTAB. There seems to be little difference between using 0.2 or 0.4% (w/v) CTAB in terms of extraction of the HOX enzyme.

Table 8

Extraction of HOX from *Hansenula polymorpha* with various quaternary ammoniumcompounds 5

| Trade name | [a]Methylene groups in chain | Counter ion | Extracted HOX activity normalised with extracted amount with LTAB | [b]standard deviation |
|---|---|---|---|---|
| LTAB | 11 | bromide | 100 | 7 |
| Cetrimide-40 | 13 | bromide | 62 | 6 |
| Cetrimide-40 dissolved in butanol | 13 | bromide | 65 | 1 |
| CTAB | 15 | bromide | 53 | 10 |
| STAB | 17 | bromide | 38 | 11 |
| MTAC | 13 | chloride | 71 | 2 |
| CTAC | 15 | chloride | 67 | 7 |
| STAC | 17 | chloride | 54 | 10 |
| *Pichia pastoris* LTAB | 11 | bromide | 3000[c] | not determined |
| *Pichia pastoris* Control | - | bromide | 100[c] | not determined |

The extracellular HOX levels in the fermentation broth before addition of extraction reagents was about 9% of the HOX activity extracted with LTAB after 24 h.
[a]The compounds are all of the structure: $CH_3-(CH_2)_n-N(CH_3)^+3$ with chloride or bromide as counter ion.
[b]All experiments were performed in duplicate
[c]The results from *Pichia pastoris* were normalised with respect to extracted HOX in the control tube without any LTAB added. The extracellular HOX level in the fermentation before start of the extraction was about 24% of the extracted level in the control, i.e. the plastic tube without any LTAB added.

Table 9

Time course of extraction of the HOX enzyme with CTAB, LTAB, and CTAC

| | 0.4% (w/v) | | | 0.2% (w/v) | |
|---|---|---|---|---|---|
| Time [h] | CTAB | LTAB | CTAC | CTAB | LTAB |

(continued)

Time course of extraction of the HOX enzyme with CTAB, LTAB, and CTAC

| | 0.4% (w/v) | | | 0.2% (w/v) | |
|---|---|---|---|---|---|
| 0 | 3±1, n=3 | 6±1, n=3 | 4±1, n=3 | 2 | 5 |
| 7 | 9 | 25 | 8 | 8 | 15 |
| 17 | 28 | 74 | 27 | 38 | 49 |
| 24 | 36±5, n=3 | 83±8, n=3 | 38±6, n=3 | 43 | 65 |
| 48 | 65±8, n=3 | 100±25, n=3 | 64±20, n=3 | 65 | 78 |

The extracellular HOX level in the fermentation broth before addition of extraction reagents was about 4% of the HOX activity extracted with 0.4% (w/v) LTAB after 48 h. Values are given ±1 standard deviation. n: the number of experiments.

All values are normalised to the extracted levels with 0.4% (w/v) LTAB after 48h.

**Example 10**

**Comparison between CTAB and other emulsifiers for extraction of HOX**

It is known that lysolecithin (lysophosphatidylcholine) can permeabilize at least mammalian cells, with selective release of macromolecules. In order to test the effects of lysolecithin and a number of other emulsifiers and short chain fatty acids, their ability to extract HOX was examined and compared with CTAB.

Five mL of cell suspension (cells + supernatant) was added to a 15 mL centrifuge tube (HOX9910B, 305 g cells/litre wet weight, 1.6 U/ml extracellular HOX activity). Cells were separated by centrifugation at 4000 g for 10 min. Cells were then re-suspended in 4.0 mL 25 mM citric acid, pH 6.3 supplemented with either CTAB, emulsifiers SLL, YN, capric acid, lysolecithin, or lecithin. The cells were then incubated for 20 hours at 25°C (500 rpm).

**Results 10**

After incubation, the level of HOX activity in the cell free extract was measured by the HOX assay. The data presented in Table 10 indicate that the tested emulsifiers other than CTAB are only capable of releasing very low levels of active enzyme. The results also indicate that CTAB is capable of activating latent enzyme in the supernatant, possibly by releasing the enzyme from membrane bound fragments.

Table 10

Effect of detergent, Emulisifier and Phospho-lipids on the extraction of HOX

| Test | HOX activity, % |
|---|---|
| Control (Cells and buffer) | 100 |
| 0.4% CTAB | 1100 |
| 0.5% emulsifier SSL | 160 |
| 1.0% emulsifier SSL | 140 |
| 0.5% emulsifier YN | 130 |
| 1.0% emulsifier YN | 130 |
| 0.5% capric acid | 120 |
| 1.0% capric acid | 140 |
| 0.4% lyso-lecithin | 260 |
| 0.4% lecithin | 140 |
| Control (Cells + supernatant) | 170 |
| Cells +supernatant+ 0.4% CTAB | 1900 |

**Example 11**

**Comparison between CTAB and saponin for extraction of HOX**

In order to establish whether saponin works like digitonin, the effect of saponin on the extraction of the HOX

enzyme from *Hansenula* was examined.

The experiment was carried out with 5.0 mL cell suspension (cells + supernatant) in a 15 mL centrifuge tube (HOX190799, 340 g cells/litre wet weight, 0.5 U/mL extracellular HOX activity). Cells were separated by centrifugation at 4000 g for 10 min. The cells were then re-suspended in 4.0 mL of supernatant which was supplemented with either CTAB, or saponin, or re-suspended in 25 mM citric acid, pH 6.3 supplemented with either CTAB, or saponin.

In order to confirm that the measured HOX activity in the cell-free extract (after treatment with CTAB) is actually the result of extraction and not just the result of HOX activation in the supernatant (it could be that HOX already exists in the supernatant but is inactive), the cell-free supernatant (after supplementation with CTAB or saponin) was also incubated and analysed for HOX activity. The tubes were incubated for 19 hours at 25˚C (500 rpm). After incubation, the extracellular HOX in the cell-free extract was measured by the HOX assay.

**Results 11**

The results in Table 11 show that saponin has a negligible ability to extract HOX from the cells. In addition, there is no indication of HOX activation neither by saponin nor by CTAB.

Table 11

Comparative HOX extraction/activation by using different permeabilising agents

| Test | HOX activity, % |
|---|---|
| Control 0 (cells + supernatant) | 100 |
| 0.2% CTAB (cells + supernatant) | 1200 |
| 0.4% CTAB (cells + supernatant) | 3100 |
| 0.2% Saponin (cells + supernatant) | 150 |
| 0.4% Saponin (cells + supernatant) | 140 |
| 0.8% Saponin (cells + supernatant) | 140 |
| Control 1 (cells + buffer) | 100 |
| 0.2% CTAB (cells + buffer) | 3100 |
| 0.4% CTAB (cells + buffer) | 7700 |
| 0.2% Saponin (cells + buffer ) | 230 |
| 0.4% Saponin (cells + buffer ) | 230 |
| 0.8% Saponin (cells + buffer ) | 230 |
| Supernatant + 0.2% CTAB | 80 |
| Supernatant + 0.4% CTAB | 80 |
| supernatant + 0.2% Saponin | 80 |
| Supernatant + 0.4% Saponin | 80 |
| Supernatant + 0.8% Saponin | 80 |

**Example 12**

**CTAB extraction of HOX in 100 L fermentor**

After 120 h of fermentation (FermID Vest9910b) a CTAB solution (360 g CTAB dissolved in 3.6 L water at 40˚C) was added directly to the broth through an inlet port in the 100 L fermentor. The final concentration of CTAB in the fermentation broth was approximately 4 g/L, since the active fermentor volume was approximately 90 L.

Simultaneously, agitation, aeration, pH control and feed addition were stopped. The temperature was controlled to 25˚C, and after 22 h of CTAB treatment the broth's HOX content had increased from 1.6 U/mL to 30 U/mL.

**Example 13**

**Homogenization of HOX producing *Hansenula polymorpha* in lab scale**

In order to test the efficiency of HOX extraction as a result of the CTAB treatment, the cells from two different fermentation trials were disrupted by using a cell disruption equipment "Z Plus" 2.2 kW (Constant Systems Ltd, UK).

The cells (5 mL) were disrupted using a one shot pump head at various pressures. After opening, the cell debris was separated from the supernatant by centrifugation (5 min at 10,000 g) and the intracellular HOX level in the cell-free supernatant was measured using the HOX assay as previously described. The same cells have also been treated with 0.2% CTAB (25˚C, 500 rpm, 20h) and cell-free extract was used as a comparable matter.

**Results 13**

The data presented in Table 12 indicate that the total amount of intracellular HOX is extracted by treatment with 0.2% CTAB.

Table 12

Efficiency of CTAB-treatment

| Test | Pressure [bar] | HOX activity [U/mL] |
|---|---|---|
| HOX9931-8 | 1500 | 14.1 |
| HOX9931-8 | 2000 | 16.3 |
| HOX9931-8 | 2200 | 16.4 |
| HOX9931-8 | 2500 | 16.2 |
| HOX9931-8 | 2600 | 16.7 |
| HOX9931-8 | 2700 | 15.7 |
| HOX9931-8+0.2% CTAB* | - | 18.6 |
| HOX9934-8 | 2700 | 8.9 |
| HOX9934-8+0.2% CTAB* | - | 7.3 |

* Cells were incubated for 48 hours at 25˚C

**Example 14**

**Homogenization of HOX producing *Hansenula polymorpha* in large scale**

10 L of fermentation broth (FermID Vest9907b) was homogenised in an APV Gaulin high pressure homogenizer model 30 CD. The homogenizer was operated by maximum flow rate (100 L/min) and by a pressure of 1000 bar. During the homogenisation procedure the broth was cooled with ice water, and the product temperature never exceeded 20˚C. A rapid increase in HOX activity was observed during the first three cycles, followed by an almost steady level after 5-7 cycles.

**Results 14**

The results are shown in Table 13 and in Figure 8.

Table 13

Mechanical extraction of HOX from *Hansenula polymorpha*

| Cycle # | HOX activity [U/mL] |
|---|---|
| 0 | 0.86 |
| 1 | 5.6 |
| 2 | 6.4 |
| 3 | 6.6 |
| 4 | 6.6 |
| 5 | 6.7 |
| 6 | 6.9 |
| 7 | 6.7 |

**Example 15**

**The effect of Triton X-100 on the extraction of HOX from *Hansenula polymorpha***

CTAB or Triton X-100 was added to 5 mL of ferment, (sample HOX9954, Mut 45, 18.10.99, HVP) in a centrifuge tube.. Water was added to the control. The samples were incubated at 25°C for 22h at 200 rpm. After incubation the samples were centrifuged and the supernatant was analyzed for HOX activity as previously described.

**Results 15**

The results are shown in Table 14 and Figure 9. The non-ionic detergent, Triton X-100 has been used to permeabilize yeast cells (see Naglak *et al* 1990 and US Patent No 5124256) but it is clear from this experiment that Triton X-100 has no extracting effect, contrary to CTAB which has not been described in the prior art to be capable of extracting an intracellular enzyme such as a HOX enzyme, although it has been described to give permeabilisation of cells.

Table 14
HOX extraction with CTAB compared to Triton X-100

| Test | HOX activity [U/mL] |
|---|---|
| 0.2% CTAB | 14.5 |
| 0.4% CTAB | 20.5 |
| 0.1% Triton X-100 | 1.5 |
| 0.2% Triton X-100 | 1.6 |
| 0.4% Triton X-100 | 1.8 |
| 0.6% Triton X-100 | 1.9 |
| 1.0% Triton X-100 | 1.9 |
| Control, ferment | 1.2 |

**Example 16**

**Western blotting**

Western blotting was used to test the efficiency of HOX secretion by analysing the amounts of residual (pellet) and released (supernatant) HOX enzyme. Cells were treated with 0, 0.1, 0.2 and 0.4% CTAB, respectively, for 20 hours. After incubation the cells were separated by centrifugation at 4000 g for 10 min. SDS-Page (4-12% Mes NuPage) of the resultant supernatant is shown in lane 7-10 of Figure 10A. The pellets were washed twice with buffer, and then re-suspended in buffer and disrupted on a FastPrep cell disrupter. The pellet extracts were also applied to an SDS-PAGE (see lanes 2-5 in Figure 10A), using precast Novex gels according to manufacturer's instructions (Novex, San Diego, US). The SDS-Page gel was blotted to a nitrocellulose membrane according to manufacturer's instructions (Novex, San Diego, US). The blot was incubated with antibodies (rabbit antiserum # 4364 BI/OCH 190797) raised against the HOX enzyme, the preparation of which is described below.

**Production of HOX specific antibodies**

A recombinant HOX enzyme was produced in *Escherichia coli* from the expression plasmid PUPO181 as described in WO 96/40935. The crude extract of *E. coli* cells expressing recombinant HOX was analysed by SDS-PAGE. A prominent protein band at the relative molecular weight (Mr) of 62 kD corresponding to HOX was transferred to a polyvinylidene difluoride (PVDF) membrane and subjected to N-terminal amino acid sequence analysis as described in WO 96/40935. The amino acid sequence identified was: Ala-Thr-Leu-Pro-Gln-Lys-Asp-Pro-Gly-Tyr- (SEQ ID NO: 1). This sequence corresponded to amino acids Nos. 2-11 in the published sequence for HOX (Hansen and Stougaard, 1997). Therefore, it was concluded that the expressed 62 kD protein was recombinant HOX lacking the N-terminal amino acid methionine, $Met_1$.

The 62 kD HOX band observed in SDS-PAGE was purified by preparative SDS-PAGE and electroelution from the gel as described by Hunkapiller *et al* (1983). The purity of the electroeluted 62 kD HOX band was analysed by SDS-PAGE and by amino acid sequence analysis as described above. The purified HOX was used for antibody production in rabbits. Portions of approximately 50 µg were mixed with an equal volume of incomplete Freund's adjuvant and used

for immunization.

The HOX specific polyclonal antibodies produced in the rabbits were used throughout this study in Western blot analyses. Proteins to be analysed by Western blot analysis were electrophoresed as described above and transferred to a nitrocellulose filter according to standard procedures. The nitrocellulose membrane was blocked 1 hour in a TBS-T solution (50 mM Tris, pH 7.5; 150 mM NaCl; O.1 % Tween-20) containing 3% skimmed milk powder. HOX specific antibodies diluted 1:10,000 in TBS-T containing 1.5% skimmed milk powder were added and the blot was incubated overnight. The blot was washed three times in TBS-T before incubation (1 to 2 hours) with the secondary antibody (alkaline phosphatase-conjugated goat anti-rabbit immunoglobulins, DAKO, cat. no. D0487), diluted 1: 1000 in TBS-T containing 1.5% skimmed milk powder. The blot was subsequently washed in TBS-T (2 x 20 min) and in TBS (50 mM Tris, pH 7.5; 150 mM NaCl; 1 x 5 min) before development in Nitroblue tetrazolium/5-Bromo-4-chloro-3-indolylphosphate (NBT/BCIP) buffer according to standard procedures.

The specificity of the antibodies was investigated in a series of Western blots with HOX containing extracts from *Chondrus crispus, E. coli* and *Pichia pastoris,* respectively. Western blot analysis of HOX containing extracts of *P. pastoris* showed a strong HOX specific band at Mr 62 kD in addition to two or three weaker bands at lower molecular weight.

**Results 16**

The results of the Western blot are shown in Figure 10B. This Western blot confirms that practically no HOX is left in the cells after treatment with 0.4% CTAB.

**Example 17**

**Description of High Throughput Screening (HTS) for increased levels of intracellular enzymes**

A *Hansenula polymorpha* strain expressing the intracellular HOX enzyme was mutated with UV light at a wavelength of 254 nm. The mutated strain was plated on agar plates (1.4 g/L Yeast Nitrogen Base (YNB) from Gibco, 5 g/L $(NH_4)_2SO_4$, 1 g/L glycerol and 2% (w/v) agar) and incubated at 30˚C until colonies were formed. The colonies were inoculated with a robotic colony picker (Q-Pix, Genetix, Christchurch Dorsett, UK) into 96 well microtiter plates. Each microtiter well contained 200 μL YNB medium (100 mM MES pH 6.1, 1.4 g/L YNB from Gibco, 5 g/L $(NH_4)_2SO_4$ and 10 g/L glycerol). The microtiter plates were incubated at 25˚C with shaking for 7 days in an IOC400.XX2.C shaking incubator (SANYO Gallenkamp BV, Breda, The Netherlands). HOX activities were measured on 10 μL fermentation broth with the HOX assay modified to contain only 105 μL reagent 1 and 15 μL 0.4% (w/v) CTAB was added to the assay. The reaction time was 60 minutes at 30˚C. The HOX assay was carried out with a Plato 7 pipetting robot (Rosys, Hombrechtikon, Switzerland) and the absorbencies were measured in a Spectramax plus microtiter plate reader (Molecular Devices, UK). The growth in each individual microtiter well was measured by transferring 10 μL of fermentation broth to a new microtiter plate, adding 100 μL of 100 mM phosphate buffer, pH 6.3 and measuring the absorbency at 600 nm. The HOX measurements were normalized with respect to the absorbency at 600 nm to take poor growth into account.

**Results 17**

The results demonstrate that it is possible to screen for mutants of *Hansenula polymorpha* producing elevated levels of intracellular HOX enzyme.

**Example 18**

Comparison of specific activity from CTAB extracted HOX (see for example Table 12) and "mechanically extracted" HOX enzymes (see for example Table 13 and Figure 8).

**Results 18**

The results demonstrate that the specific activity of CTAB extracted HOX is higher than the specific activity of "mechanically extracted" HOX. These results indicate that the CTAB does not extract all of the intracellular proteins localised in the organelle, but mainly the cytosolic proteins.

**Example 19**

**Characterisation of CTAB- and mechanically extracted HOX by anion exchange chromatography**

In order to analyse the purity of the CTAB extracted HOX, it was compared to HOX extracted by using cell disruption. The specific activity was determined and compared, and the nucleic acid contents of the extracts were compared. Furthermore the purity was examined by anion exchange chromatography.

Seven mL cell suspension (cell + supernatant) was added to a 15 mL centrifuge tube (HOX9957, Mut 45). Upon addition of 0.4% CTAB the cell suspension was incubated for 23 hours at 30˚C (200 rpm). The cells were removed by centrifugation (10000 g and 10 min) and cell-free supernatant was used as a source of CTAB extracted HOX. Another 7 mL of the same cell suspension (without adding CTAB) was disrupted by using a one shot pump head at 2 X 2400 bar (Z Plus, 2.2 kW, Constant Systems Ltd, UK). The cell debris was then separated by centrifugation (10000 g and 10 min) and the supernatant was used as a source of mechanically extracted HOX.

Both samples were desalted on a PD10 column (Pharmacia Biotech.) in 20 mM TEA (triethanolamin, Merck) buffer, pH 7.3. The samples were analysed for HOX activity and protein-concentration (protein assay is based on the assay method described by Schleif and Wensink, 1981. The nucleic acid content was determined by measurement of the absorption at 260 and 280 nm (Bollag and Edelstein, 1991.

Ion Exchange Chromatography was carried out by using a Biologic Duo Flow (BioRad, CA, USA) system. 500 μl of desalted sample was applied to a Source Q 15 column (HR5/5, Pharmacia Biotech.) equilibrated in TEA buffer (buffer A, 20 mM, pH 7.3). The HOX was eluted with a 20 mL linear gradient from 0-0.5 M NaCl in buffer A with a flow rate of 1.5 mL/min during which 1.5 mL fractions were collected and assayed for HOX activity.

**Result 19**

Determination of specific activity shows that CTAB extracted HOX is much more pure compared to mechanically extracted HOX (Table 15). Also the nucleic acid content is much lower in the CTAB extracted HOX than in the mechanically extracted HOX (Table 15).

Table 15

HOX- and protein concentration in CTAB- and mechanically extracted HOX

| Test | HOX activity [U/mL] | Protein concentration [mg/mL] | Specific activity [U/mg protein] | Nucleic acid concentration [μg/mL] |
|---|---|---|---|---|
| CTAB extracted | 30.6 | 2.33 | 13.1 | 102 |
| Mechanically extracted | 32.0 | 12.7 | 2.5 | 384 |

The anion exchange chromatography analyses in Figures 11A and 11B which show chromatograms of the Source Q analyses for the CTAB- and mechanically extracted HOX also strongly confirm this result.

**Experiments with CTAB**

**Example 20**

**1. shake flask experiments**

Two different media were chosen for experiments with CTAB:

- YP/1 % glycerol
- YNB/1 % glycerol + 0.1 M NaPi pH 6.0

**a) cultivation in YP/1 % glycerol**

50 mL medium was inoculated with 2.5 mL of a YPD preculture and cultivated at 37˚C, 160 rpm.

After 28 h cultivation, 1 % (v/v) methanol was added and further incubated for 18 h at 37˚C, 160 rpm.

The $OD_{600nm}$ was measured to calculate the amount of CTAB which is necassary. Aliquots of the supernatant

(SN) and the cell pellet of 1.5 mL culture were taken. After mechanical disruption of the cells the soluble fraction (CX) was isolated.
=> SN of these conditions was designated A
CX of these conditions was designated D

Same volumes of the culture (20 mL) were aliquoted into two shake flasks. 20 mL of the culture was supplemented with 0.005 g CTAB.
(CTAB - stock solution: 0.02 g/mL; DANISCO: 0.4 % in fermentor culture ($OD_{600nm}$ ~ 300)
=> shake flask experiments $OD_{600nm}$ ~ 20 => 0.027 g CTAB /100 mL culture) incubation of the culture: 24 h, 4°C without shaking
=> SN of these conditions was designated C CX of these conditions was designated F

The second shake flask without CTAB was incubated under the same conditions as the CTAB - flask and served as reference culture.
=> SN of these conditions was designated B
CX of these conditions was designated E

Strains, harbouring five different IL-1ra constructions were cultivated. The strains 4-17, AL 9/2 and II 3-1 contained three different constructions without a signal sequence, while the strains MFα 2 and MFα AL7/1 represented two different constructions with the MFα pre-pro sequence.

Strain FPMT 8 was cultivated under the same conditions as the recombinant strains. This strain is an RB11 integrant with nearly 30 copies of the empty *Hansenula* vector pFPMT121 and served as negative control.

After treatment with CTAB, a 40fold (20fold) to 110fold increase of the IL-1ra concentration was detected in the supernatant of strains, harbouring constructions without signal sequences.

For the MFα- strains treated with CTAB, a lower increase (2 to 5fold) of the IL-1ra concentration was measured.

**Results 20**

The results are summarized in table 16

*Table 16:* experiments with CTAB in YP/ glycerol/methanol

| strain | sequence | $OD_{600nm}$ | SN sample | ELISA IL-1ra [μg/mL] | factor |
|---|---|---|---|---|---|
| 4-17 | 2 | 20,5 | A<br>B<br>C | 0,345<br>0,346<br>39,0 | C/A= 113<br>CB= 113 |
| AL9/2 | 3 | 22,6 | A<br>B<br>C | 0,166*<br>0,179<br>3,39 | C/A = 20<br>C/B=19 |
| | | 18,7 | A | 1,67 | C/A = 49 |
| II 3/1 | 4 | | B<br>C | 1,94<br>81,2 | C/B=42 |
| MF α2 | 6 | 20,4 | A<br>B<br>C | 4,85<br>5,69<br>27,7 | C/A = 6<br>CB=5 |
| MF αAL 7/1 | 8 | 22,6 | A<br>B<br>C | 2,28<br>2,02<br>5,23 | C/A = 2,3<br>C/B = 2,6 |

A: supernatant after cultivation for 46 h in YP/glycerol/methanol
B: supernatant after cultivation for 46 h in YP/glycerol/methanol, than incubated for 24 h without CTAB
C: sterile filtrated supernatant after cultivation for 46 h in YP/glycerol/methanol, than incubated for 24 h with CTAB
***** remarks:

The IL-1ra concentration in supernatant of strain AL 9/2 was unusually low. In furth experiments concentrations between 0.6 and 0.7 μg/mL were detected.
The reason for the low yield is not known.

**Example 21**

**b) cultivation in YNB/1 % glycerol + 0.1 M Na Pi pH 6.0**

For further experiments with CTAB, three strains harbouring three different constructs without signal sequences were selected (strain 4-17; AL 9/2; II 3-1).

45 mL medium was inoculated with 5 mL of a YPD preculture and cultivated at 37°C, 160 rpm.

After 28 h cultivation 1 % (v/v) methanol was added and further incubated for 18 h at 37°C, 160 rpm.

The $OD_{600nm}$ was measured to calculate the amount of CTAB which is necessary. Aliquots of the supernatant (SN) and the cell pellet of 3 mL culture were taken. After mechanical disruption of the cells the soluble fraction (CX) was isolated.

=> SN of these conditions was designated A

CX of these conditions was designated D

Same volumes of the culture (20 mL) were aliquoted into two shake flasks. 20 mL of the culture was supplemented with 0.003 g CTAB. incubation of the culture: 24 h, 4°C without shaking

=> SN of these conditions was designated C

CX of these conditions was designated F

The second shake flask without CTAB was incubated under the same conditions as the CTAB - flask and served as reference culture.

=> SN of these conditions was designated B

CX of these conditions was designated E

In all cases, incubation with CTAB led to an significant increase of the IL-1ra concentration in the supernatant (100 to 130fold).

**Results 21**

The ELISA results of the CTAB experiments after cultivation in two different media are compared in the following table 17.

*Table 17:* comparison of CTAB experiments in YP/glyc/methanol und YNB/glyc/methanol

| | | **YP/glyc/methanol** | | | **YNB/glyc/methanol (pH 6,0)** | | |
|---|---|---|---|---|---|---|---|
| **strain** | **SN sample** | **$OD_{600nm}$** | **ELISA IL-1ra [μg/mL]** | **factor** | **$OD_{600nm}$** | **ELISA IL-1ra [μg/mL]** | **factor** |
| 4-17 | A | 20,5 | 0,345 | C/A = 113 | 10,2 | 0,205 | C/A = 108 |
| | B | | 0,346 | C/B = 113 | 10,8 | 0,069 ? | (C/B = 322) |
| | C | | 39,0 | | 9,8 | 22,2 | |
| AL 9/2 | A | 22,6 | 0,166 | C/A = 20 | 10,1 | 0,045 | C/A = 137 |
| | B | | 0,179 | C/B=19 | 11,6 | 0,025 ? | (C/B = 246) |
| | C | | 3,39 | | 11,0 | 6,16 | |
| II 3/1 | A | 18,7 | 1,67 | C/A = 49 | 10,0 | 0,276 | C/A = 105 |
| | B | | 1,94 | C/B = 42 | 11,4 | 0,279 | C/B = 104 |
| | C | | 81,2 | | 10,6 | 29,1 | |

A: supernatant after cultivation for 46 h in YP/glycerol/methanol (or YNB/glycerol/methanol)

B: supernatant after cultivation for 46 h in YP/glycerol/methanol (or YNB/glycerol/methanol), than incubated for 24 h without CTAB

C: steril filtrated supernatant after cultivation for 46 h in YP/glycerol/methanol (or YNB/glycerol/methanol), than incubated for 24 h with CTAB

**Example 22**

**Test of different incubation conditions**

For strain II 3/1, cultivated in YP/glycerol/methanol (see 1.a) different incubation conditions after addition of CTAB were tested.

conditions:

- ➢ 24 h CTAB, 4˚C without shaking ("standard" condition)
- ➢ 24 h CTAB; 4˚C gently shaking
- ➢ 24 h CTAB, 37˚C without shaking
- ➢ 24 h CTAB; 37˚C gently shaking

**Results 22**

The concentration of IL-1ra in the supernatant was measured by ELISA. The results are summarized in table 18.

*Table 18*:different incubation condition of CTAB (ELISA results)

| strain II 3/1 | ELISA IL-1ra [μg/mL] | factor |
|---|---|---|
| supernatant A | 1,67 | |
| 4°C without shaking } B C | 1,94<br>81,2 | C/B=42<br>C/A = 49 |
| 4°C gently shaking } B C | 1,62<br>44,7 | C/B = 28<br>C/A = 27 |
| 37°C without shaking } B C | 8,04<br>127,4 | C/B=16<br>C/A = 76 |
| 37°C gently shaking } B C | 11,1<br>46,2 | C/B= 4 C/A = 28 |

A: supernatant after cultivation for 46 h in YP/glycerol/methanol
B: supernatant after cultivation for 46 h in YP/glycerol/methanol, than incubated for 24 h without CTAB
C: steril filtrated supernatant after cultivation for 46 h in YP/glycerol/methanol, than incubated for 24 h with CTAB

The highest increase of IL-1ra in the supernatant was measured after CTAB incubation at 37˚C without shaking (76fold) and after CTAB incubation at 4˚C without shaking (49fold).

The highest IL-1ra concentration was detected at 37˚C, but the concentration in the reference sample incubated without CTAB was also increased (16 fold). The high concentration in the reference sample could be caused by cell lysis.
=> best conditions: 4˚C (to avoid cell lysis) without shaking

**Example 23**

**SDS-PAGE, western blot and coomassie staining**

The supernatant and the soluble fraction of the crude extract isolated from the shake flask experiments were

analyzed by SDS-PAGE under reducing conditions.

gel: 16 % Novex-gel TG 1mm; reducing conditions colloidal coomassie staining (BIO-SAFE Coomassie, Biorad)

samples: A: supernatant after cultivation for 46 h in YP/glycerol/methanol
B: reference supernatant without CTAB
D: soluble fraction (CX) of crude extract **1:3 diluted**
E: soluble fraction (CX) of crude extract reference culture **1:3 diluted**

F: soluble fraction (CX) of crude extract after CTAB treatment **1:3 diluted**

**Results 23**

❖ **WB 33 and Coo2**

strains: 4-17 pFPMT icIL 1raI
A19/2pFPMT icIL 1raI + AI

CTAB: incubation for 24 h; 4˚C without shaking

The western blot (WB 33) results are presented in **Figure 12A**

The test samples and quantities addded are presented in the following legend to Figure 12A.

| | | | |
|---|---|---|---|
| 1. MW marker See Blue (total) | | | 10 µL |
| 2.4.17 | **A** SN | | 11,3 µL |
| 3.4-17 | **D** CX | 1:3 dil. | 11,3 µL |
| 4.4-17 | **C** SN CTAB | | 11,3 µL |
| 5. 4-17 | **F** CX CTAB | 1:3 dil. | 11,3 µL |
| 6. rhIl-lra-standard (BSA-free) | | | 30 ng |
| 7. AL 9/2 | **A** SN | 11,3 µL | |
| 8. AL 9/2 | **D** CX | 1:3 dil. | 11,3 µL |
| 9. AL 9/2 | **C** SN CTAB | | 11,3 µL |
| 10. AL 9/2 | **F** CX CTAB | 1:3 dil. | 11,3 µL |

The results demonstrate that for both strains an increase of IL-1ra in the SN (lane 4, lane 9) and a decrease in the CX (lane 5, lane 10) was detected after treatment with CTAB.

The colloidal coomassie (Coo 2) blue staining is shown in **Figure 12B** The test samples and quantities addded are presented in the following legend to Figure 12B.

| | | | |
|---|---|---|---|
| 1. MW marker Mark 12 10 µL (total) | | | |
| 2.4-17 | **A** SN | | 11,3 µL |
| 3.4-17 | **D** CX | 1:3 dil. | 11,3 µL |
| 4. 4-17 | **C** SN CTAB | | 11,3 µL |
| 5. 4-17 | **F** CX CTAB | 1:3 dil. | 11,3 µL |
| 6. 4-17 | **B** SN w/o CTAB | | 11,3 µL |
| 7. 4-17 | **E** CX w/o CTAB | 1:3 dil. | 11,3 µL |
| 8.rhIl-1ra-Standard (BSA-free) | | | 100 ng |

(continued)

| | | | |
|---|---|---|---|
| 1. MW marker Mark 12 10 μL (total) | | | |
| 9. AL 9/2 | **A** SN | | 11,3 μL |
| 10. AL 9/2 | **D** CX | 1:3 dil. | 11,3 μL |
| 11. AL 9/2 | **C** SN CTAB | | 11,3 μL |
| 12. AL 9/2 | **F** CX CTAB | 1:3 dil. | 11,3 μL |
| 13. AL 9/2 | **B** SN w/o CTAB | | 11.3 μL |
| 14. AL 9/2 | **E** CX w/o CTAB | 1:3 dil. | 11,3 μL |
| 15. FPMT 8 | **A** SN | | 11,3 μL |

❖ **WB 34 and Coo 3**

strains: MF α2 pFPMT MFα IL-1raI
MFαAL 7/1pFPMT MFα IL-1raI + AI
CTAB: incubation for 24 h; 4˚C without shaking

The western blot (WB 34) results are presented in **Figure 13A**
The test samples and quantities addded are presented in the following legend to Figure 13A.

| | | | |
|---|---|---|---|
| 1. MW marker | See Blue | 10 μL (total) | |
| 2. MFα 2 | **A** SN | | 11,3 μL |
| 3. MFα 2 | **D** CX 1:3 dil. | | 11,3 μL |
| 4. MFα 2 | **C** SN CTAB | | 11,3 μL |
| 5. MFα 2 | **F** CX CTAB | 1:3 dil. | 11,3 μL |
| 6. rhIl-1ra-standard (BSA-free) | | | 30 ng |
| 7. MFα AL7/1 | **A** SN | | 11,3 μL |
| 8. MFα AL7/1 | **D** CX | 1:3 dil. | 11,3 μL |
| 9. MFα AL7/1 | **C** SN CTAB | | 11,3 μL |
| 10. MFα AL7/1 | **F** CX CTAB | 1:3 dil. | 11,3 μL |

The results indicate that after treatment with CTAB a mixture of intracellular and secreted IL-1ra was detected in the supernatants C in lane 4 and 9.
MFα 2: additional band of ~ 20 kDa and 34 kDa derived from intracellular IL-1ra
MFα 7/1: additional band of < 17 kDa derived from intracellular IL-1ra intensity of 18 kDa signal increased
The colloidal coomassie (Coo 3) results are presented in **Figure 13B**
The test samples and quantities addded are presented in the following legend to Figure 13B.

| | | | |
|---|---|---|---|
| 1. MW marker Mark 12 | | | 10 μL (total) |
| 2. MFα 2 | **A** SN | | 11,3 μL |
| 3. MFα 2 | **D** CX | 1:3 dil. | 11,3 μL |
| 4. MFα 2 | **C**. SN CTAB | | 11,3 μL |
| 5. MFα 2 | **F** CX CTAB | 1:3 dil. | 11,3 μL |
| 6. MFα 2 | **B** SN w/o CTAB | | 11,3 μL |
| 7. MFα 2 | **E** CX w/o CTAB | 1:3 dil. | 11,3 μL |
| 8.rhIl-1ra-Standard (BSA-free) | | | 100 ng |
| 9. MFα AL7/1 | **A** SN | | 11,3 μL |
| 10. MFα AL7/1 | **D** CX | 1:3 dil. | 11,3 μL |
| 11. MFα AL7/1 | **C** SN CTAB | | 11,3 μL |
| 12.. MFα AL7/1 | **F** CX CTAB | 1:3 dil. | 11,3 μL |
| 13. MFα AL7/1 | **B** SN w/o CTAB | | 11,3 μL |

(continued)

| | | | |
|---|---|---|---|
| 1. MW marker Mark 12 | | | 10 μL (total) |
| 14. MFα AL7/1 | **E** CX w/o CTAB | 1:3 dil. | 11,3 μL |
| 15. FPMT 8 | **C** SN CTAB | | 11,3 μL |

❖ **WB 35 and Coo 4**

strain:II 3/1 pFPMT icIL-1ra type II
different incubation conditions after addition of CTAB:

- ➢ 24 h CTAB, 4˚C without shaking ("standard" condition)
- ➢ 24 h CTAB, 37˚C without shaking

The western blot (WB 35) results are presented in **Figure 14A**

The test samples and quantities addded are presented in the following legend to Figure 14A.

| | | | |
|---|---|---|---|
| 1. MW marker See Blue | | | 10 μL (total) |
| 2. II 3/1 | SN | | 11,3 μL |
| 3. II 3/1 | CX | 1:3 dil. | 11,3 μL |
| 4. II 3/1 | SN CTAB 4˚C | | 11,3 μL |
| 5. II 3/1 | CX CTAB 4˚C | 1:3 dil. | 11,3 μL |
| 6. rhIl-1ra-Standard (BSA-free) | | | 30 ng |
| 7.II 3/1 | SN CTAB 37˚C | | 11,3 μL |
| 8. II 3/1 | CX CTAB 37˚C | 1:3 dil. | 11,3 μL |
| 9. II 3/1 | SN w/o CTAB 37˚C | | 11,3 μL |
| 10. II 3/1 | CX w/o CTAB 37˚C | 1:3 dil. | 11,3 μL |

The colloidal coomassie (Coo 4) results are presented in **Figure 14B**
The test samples and quantities addded are presented in the following legend to Figure 14B.

| | | | |
|---|---|---|---|
| 1. MW marker Mark 12 | | | 10 μL (total) |
| 2. II 3/1 | SN | | 11,3 μL |
| 3.II 3/1 | CX | 1:3 dil. | 11,3 μL |
| 4. II 3/1 | SN CTAB 4˚C | | 11,3 μL |
| 5. II 3/1 | CX CTAS 4˚C | 1:3 dil. | 11,3 μL |
| 6.II 3/1 | SN w/o CTAB 4˚C | | 11,3 μL |
| 7.II 3/1 | CX w/o CTAB 4˚C | 1:3 dil. | 11,3 μL |
| 8. II 3/1 | SN CTAB 37˚C | | 11,3 μL |
| 9.II 3/1 | CX CTAB 37˚C | 1:3 dil. | 11,3 μL |
| 10. II 3/1 | SN w/o CTAB 37˚C | | 11,3 μL |
| 11. II 3/1 | CX w/o CTAB 37˚C | 1:3 dil. | 11,3 μL |
| 12. rhIl-1ra-Standard (BSA-free) | | | 100 ng |
| 13. FPMT 8 | CX CTAB 4˚C | 1:3 dil. | 11,3 μL |
| 14. FPMT 8 | SN CTAB 4˚C | | 11,3 μL |
| 15. FPMT 8 SN | | | 11,3 μL |

The results demonstrate that after CTAB incubation at 4˚C as well as at 37˚C an increase of IL-1ra in the SN (WB 35: lane 4, lane 8) and a decrease in the CX (WB 35: lane 5, lane 9) was detected.

In SN CTAB 37˚C (lane 8) the highest amount of IL-1raII was obtained. This result is in agreement with the ELISA results (see table 3).

In this supernatant not only more IL-1raII but more other proteins (> 35 kDa) were stained ( Coo 4: lane 8). This observation confirmed the assumption that a significant cell lysis took place at 37˚C as compared to 4˚C.

## DISCUSSION

The codon usage of the *Chondrus crispus* HOX gene (Stougaard and Hansen 1996, Hansen and Stougaard, 1997) was modified by replacement of the low-usage codons with those of the more frequently used codons of the *Hansenula* host organism. A transformant of the methylotrophic yeast, *Hansenula polymorpha,* expression system (developed at Rhein Biotech, Düsseldorf/Germany), containing a codon optimized HOX DNA fragment for the expression of HOX was prepared.

The codon optimisation of the gene encoding the HOX enzyme resulted in high levels of expression (in terms of high levels of enzyme activity) of the HOX enzyme in the *Hansenula polymorpha* yeast host organisms. When a signal sequence was not present the HOX enzyme was localized intracellularly. However, even when a number of different signal sequences were used in different constructs, little or no HOX activity could be measured in the extracellular medium. These results indicated that the HOX enzyme is incapable of being secreted even from host strains expressing a HOX enzyme comprising a signal sequence. Western blots also confirmed that the HOX enzyme may be localized in a membrane associated fraction even when a signal sequence was present, indicating that although there is transcription and translation of the HOX gene, the HOX enzyme was not secreted and seemed to get lodged in the secretion pathway.

The extraction of the intracellular enzymatically active HOX enzyme using the method of the present invention was compared with a traditional cell disruption method and with extraction procedures using other ionic/non ionic detergents and emulsifiers. Combinations of detergents with protease and salts were also investigated.

## SUMMARY

In one broad aspect there is described herein of the present invention a method is provided for releasing a soluble or membrane associated intracellular protein of interest (POI) comprising the steps of: providing a cell comprising a soluble or membrane associated intracellular POI; contacting the cell with a membrane extracting composition; and causing the POI to be released from the cell under conditions sufficient for the release of the POI and in a soluble form.

In another broad aspect there is described herein a method is provided for specifically releasing a soluble or membrane associated intracellular protein of interest (POI) comprising the steps of: providing a cell comprising a soluble or membrane associated intracellular POI; contacting the cell with a membrane extracting composition; and causing the POI to be released from the cell under conditions sufficient for the release of the POI but insufficient for the release of other contaminating proteins.

Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## REFERENCES

Bollag, D.M. and Edelstein, S.J. (1991) Protein Methods. New York, Wiley-Liss.

Crahay, J., Delcour, J.M.A.G. and Hanotier, J.D.V. (1992) Process for recovering polypeptides localized in the periplasmic space of yeast without breaking the cell wall by using an non-ionic detergent and a neutral salt. US5124256.

Craig, W.S. (1987) Purification of pichia produced lipophilic proteins. US4683293.

Gowda, L.R., Bachhawat, N. & Bhat, S.G. (1991) Permeabilization of baker's yeast by cetyltrimethylammonium bromide for intracellular enzyme catalysis. Enzyme Microb. Technol. 13, 154-157.

Hansen, O.C. and Stougaard, P. (1997) Hexose oxidase from the red alga Chondrus crispus: purification, molecular cloning, and expression in Pichia pastoris. J. Biol. Chem. 272, 11581-11587.

Joshi, M.S., Gowda, L.R. and Bhat, S.G. (1987) Permeabilization of yeast cells (Kluyveromyces fragilis) to lactose by cetyltrimethylammonium bromide. Biotechnol. Lett. 9, 549-554.

Joshi, M.S., Gowda, L.R., Katwa, L.C. and Bhat, S.G. (1989) Permeabilization of yeast cells (Kluyveromyces fragilis) to lactose by digitonin. Enzyme Microb. Technol. 11,439-443.

Hagen, I.M. and Hyam, J.S. (1988) J. Cell Sci. 89,343-357.

Hunkapiller, M.W., Lujan, U., Ostrander, F., and Hood, L.E. (1983). Isolation of proteins from polyacrylamide gels for amino acid sequence analysis. Methods in Enzymology, 91: 227-236.

King, A.T., Davey, M.R., Mellor, I.R, Mulligan, B.J. and Lowe, K.C. (1991) Surfactant effects on yeast cells. Enzyme Microb. Technol. 13, 148-153.

Miyake, T. and Shiosaka, M. (1974) Process for the extraction of enzymes from microorganisms. US3801461.

Naglak, T.J., Hettwer, D.J. and Wang, H.Y. (1990) Chemical permeabilization of cells for intracellular product release. In Separation processes in biotechnology (Asenjo, J.A. ed) Vol 9, chapter 7. M. Dekker, New York.

Poulsen, C.H. and Høstrup, P.B. (1998) Purification and characterization of a hexose oxidase with excellent strengthening effects in bread. Cereal Chem. 75, 51-57.

Schleif, R.F. and Wensink, P.C. (1981) Practical Methods in Molecular Biology. New York, Springer-Verlag.

Sekhar, S., Bhat, N. and Bhat, S.G. (1999) Preparation of detergent permeabilized Bakers' yeast whole cell catalase. Process Biochem. 34, 349-354.

Stougaard, P. and Hansen, O.C. (1996) Recombinant hexose oxidase, a method of producing same and use of such enzyme. WO 96/40935.

Sullivan, J. D., and Ikawa, M. (1973) Purification and characterization of hexose oxidase from the red alga chondrus crispus. Biochem. Biophys. Acta 309,11-22.

## Claims

1. A method for extracting a soluble or membrane associated intracellular recombinant protein of interest (POI) from a bacterial, yeast or fungal cell comprising the steps of:

   (a) providing a bacterial, yeast or fungal cell comprising a soluble or membrane associated intracellular recombinant POI;
   (b) releasing the recombinant POI from the cell by contacting the cell with a membrane extracting composition comprising a quaternary ammonium compound at a concentration of between 0.05% to 0.6% by weight, under conditions sufficient for the release of the recombinant POI and in a soluble form; and
   (c) recovering the recombinant POI from the membrane extracting composition;

   in which the recombinant POI is a hexose oxidase (HOX) enzyme.

2. A method according to Claim 1 in which the bacterial, yeast or fungal cell is a transformed cell.

3. A method according to any preceding claim in which the intracellular recombinant POI is produced by recombinant DNA techniques.

4. A method according to any preceding claim, in which the cell is a yeast cell.

5. A method according to any preceding claim, in which the quaternary ammonium compound is selected from the group consisting of Lauroyl Trimethyl Ammonium Bromide (LTAB), Myristyl Trimethyl Ammonium Chloride (MTAC), Cetyl Trimethyl Ammonium Chloride (CTAC), Cetrimide, Cetyl Trimethyl Ammonium Bromide (CTAB), Stearoyl Trimethyl Ammonium Chloride (STAC), Stearoyl Trimethyl Ammonium Bromide (STAB), Benzalkonium Chloride (alkyldimethylbenzylammonium chloride), N-Cetylpyridinium Bromide (N-Hexadecylpyridinium bromide), N-Cetylpyridinium Chloride (N-Hexadecylpyridinium chloride), Benzyl Dimethyl Tetradecyl Ammonium Chloride, Benzyl Dimethyl Hexadecyl Ammonium Chloride and a combination of any two or more thereof.

6. A method according to any one of preceding claims in which the bacterial, yeast or fungal cell is contacted with the

membrane extracting composition at temperatures from 4˚C to 40˚C.

**7.** A method according to any one of preceding claims in which the bacterial, yeast or fungal cell is contacted with the membrane extracting composition at a pH of from 2.0 to 11.0.

**8.** A method for screening for mutated cells or transformed cells producing elevated levels of a soluble or membrane associated intracellular recombinant POI comprising the steps of:

(a) growing the mutated cells at 30˚C;
(b) incubating the mutated cells or transformed cells with the membrane extracting composition as defined in a method of any preceding claim;
(c) recovering the cell free medium;
(d) screening the cell free medium for elevated levels of the intracellular recombinant POI;
such that the presence of the intracellular recombinant POI in the cell free medium is indicative that the intracellular recombinant POI has been released, in which the mutated or transformed cell is a bacterial, yeast or fungal cell and in which the recombinant POI is a hexose oxidase (HOX) enzyme.

**9.** Use of a membrane extracting composition comprising a quaternary ammonium compound to extract a soluble or membrane associated intracellular recombinant POI from a bacterial, yeast or fungal cell, in which the membrane extracting composition comprises a quaternary ammonium compound at a concentration of between 0.05% to 0.6% by weight and is contacted with the bacterial, yeast or fungal cell, in which the released recombinant POI is recovered from the membrane extracting composition and in which the recombinante POI is a hexose oxidase (HOX) enzyme.

**10.** A method according to any preceding claim in which the hexose oxidase (HOX) enzyme comprises the amino acid sequence set out in Figure 6 (SEQ ID No 22).

**11.** A method according to Claim 10 in which the hexose oxidase (HOX) enzyme is encoded by a nucleotide sequence set out in Figure 6 (SEQ ID No 22).

**12.** A method according to Claim 10 or Claim 11 in which the hexose oxidase (HOX) enzyme is encoded by a nucleotide sequence capable of hybridising to the nucleotide sequence set out in Figure 6 (SEQ ID No 22).

**Patentansprüche**

**1.** Verfahren zum Extrahieren eines interessierenden löslichen oder membranassoziierten rekombinanten intrazellulären Proteins (POI) aus einer Bakterien-, Hefe- oder Pilzzelle, wobei das Verfahren die folgenden Stufen umfaßt:

(a) Bereitstellen einer Bakterien-, Hefe- oder Pilzzelle, die ein lösliches oder membranassoziiertes rekombinantes intrazelluläres POI umfaßt,
(b) Freisetzen des rekombinanten POI aus der Zelle durch Inkontaktbringen der Zelle mit einer Membranextraktionszusammensetzung, welche eine quaternäre Ammoniumverbindung in einer Konzentration von zwischen 0,05 und 0,6 Gewichts-% umfaßt, unter Bedingungen, die für die Freisetzung des rekombinanten POI ausreichend sind, und in einer löslichen Form und
(c) Gewinnen des rekombinanten POI aus der Membranextraktionszusammensetzung,

wobei das rekombinante POI ein Hexoseoxidase- (HOX-) Enzym ist.

**2.** Verfahren nach Anspruch 1, wobei die Bakterien-, Hefe- oder Pilzzelle eine transformierte Zelle ist.

**3.** Verfahren nach einem der vorangegangenen Ansprüche, wobei das rekombinante intrazelluläre POI durch rekombinante DNA-Techniken erzeugt wird.

**4.** Verfahren nach einem der vorangegangenen Ansprüche, wobei die Zelle eine Hefezelle ist.

**5.** Verfahren nach einem der vorangegangenen Ansprüche, wobei die quaternäre Ammoniumverbindung aus der Gruppe ausgewählt ist, bestehend aus Lauroyltrimethylammoniumbromid (LTAB), Myristyltrimethylammoniumchlorid (MTAC), Cetyltrimethylammoniumchlorid (CTAC), Cetrimid, Cetyltrimethylammoniumbromid (CTAB), Stearoyltrimethylammoniumchlorid (STAC), Stearoyltrimethylammoniumbromid (STAB), Benzalkoniumchlorid (Alkyldimethylbenzylammoniumchlorid), N-Cetylpyridiniumbromid (N-Hexadecylpyridiniumbromid), N-Cetylpyridiniumchlorid (N-Hexadecylpyridiniumchlorid), Benzyldimethyltetradecylammoniumchlorid, Benzyldimethylhexadecylammoniumchlorid und einer Kombination von irgendwelchen zweien oder mehreren davon.

**6.** Verfahren nach einem der vorangegangenen Ansprüche, wobei die Bakterien-, Hefe-oder Pilzzelle mit der Membranextraktionszusammensetzung bei Temperaturen von 4˚C bis 40˚C in Kontakt gebracht wird.

**7.** Verfahren nach einem der vorangegangenen Ansprüche, wobei die Bakterien-, Hefe-oder Pilzzelle mit der Membranextraktionszusammensetzung bei einem pH-Wert von 2,0 bis 11,0 in Kontakt gebracht wird.

**8.** Verfahren zum Screenen nach mutierten Zellen oder transformierten Zellen, die erhöhte Niveaus eines löslichen oder membranassoziierten rekombinanten intrazellulären POI erzeugen, wobei das Verfahren die folgenden Stufen umfaßt:

(a) Züchten der mutierten Zellen bei 30˚C,
(b) Inkubieren der mutierten Zellen oder transformierten Zellen mit der Membranextraktionszusammensetzung, wie in einem Verfahren nach einem der vorangegangenen Ansprüche definiert,
(c) Gewinnen des zellfreien Mediums,
(d) Screenen des zellfreien Mediums hinsichtlich erhöhter Niveaus des rekombinanten intrazellulären POI, so daß das Vorliegen des rekombinanten intrazellulären POI in dem zellfreien Medium anzeigt, daß das rekombinante intrazelluläre POI freigesetzt wurde, wobei die mutierte oder transformierte Zelle eine Bakterien-, Hefe- oder Pilzzelle ist und wobei das rekombinante POI ein Hexoseoxidase- (HOX-) Enzym ist.

**9.** Verwendung einer Membranextraktionszusammensetzung, welche eine quaternäre Ammoniumverbindung umfaßt, um ein lösliches oder membranassoziiertes rekombinantes intrazelluläres POI aus einer Bakterien-, Hefe- oder Pilzzelle zu extrahieren, wobei die Membranextraktionszusammensetzung eine quaternäre Ammoniumverbindung in einer Konzentration von zwischen 0,05 und 0,6 Gewichts-% umfaßt und mit der Bakterien-, Hefe- oder Pilzzelle in Kontakt gebracht wird, wobei das freigesetzte rekombinante POI aus der Membranextraktionszusammensetzung gewonnen wird und wobei das rekombinante POI ein Hexoseoxidase- (HOX-) Enzym ist.

**10.** Verfahren nach einem der vorangegangenen Ansprüche, wobei das Hexoseoxidase-(HOX-) Enzym die in Figur 6 dargestellte Aminosäuresequenz (SEQ ID NO: 22) hat.

**11.** Verfahren nach Anspruch 10, wobei das Hexoseoxidase- (HOX-) Enzym durch eine in Figur 6 dargestellte Nukleotidsequenz (SEQ ID NO: 22) codiert wird.

**12.** Verfahren nach Anspruch 10 oder Anspruch 11, wobei das Hexoseoxidase- (HOX-) Enzym durch eine Nukleotidsequenz codiert wird, die in der Lage ist, mit der in Figur 6 dargestellten Nukleotidsequenz (SEQ ID NO: 22) zu hybridisieren.

**Revendications**

**1.** Méthode pour extraire une protéine intéressante (POI) recombinante intracellulaire soluble ou associée aux membranes d'une cellule bactérienne, d'une cellule de levure ou d'une cellule fongique, comprenant les étapes consistant à :

(a) fournir une cellule bactérienne, une cellule de levure ou une cellule fongique comprenant une POI recombinante intracellulaire soluble ou associée aux membranes ;
(b) libérer la POI recombinante de la cellule en mettant en contact la cellule avec une composition d'extraction de membrane comprenant un composé d'ammonium quaternaire à une concentration de 0,05 % à 0,6 % en poids, dans des conditions suffisantes pour la libération de la POI recombinante et sous une forme soluble ; et
(c) recueillir la POI recombinante à partir de la composition d'extraction de membrane ;

dans laquelle la POI recombinante est une enzyme hexose-oxydase (HOX).

**2.** Méthode suivant la revendication 1, dans laquelle la cellule bactérienne, la cellule de levure ou la cellule fongique

est une cellule transformée.

**3.** Méthode suivant l'une quelconque des revendications précédentes, dans laquelle la POI recombinante intracellulaire est produite par des techniques d'ADN recombinant.

**4.** Méthode suivant l'une quelconque des revendications précédentes, dans laquelle la cellule est une cellule de levure.

**5.** Méthode suivant l'une quelconque des revendications précédentes, dans laquelle le composé d'ammonium quaternaire est choisi dans le groupe consistant en le bromure de lauroyltriméthylammonium (LTAB), le chlorure de myristyltriméthylammonium (MTAC), le chlorure de cétyltriméthylammonium (CTAC), le cétrimide, le bromure de cétyltriméthylammonium (CTAB), le chlorure de stéaroyltriméthylammonium (STAC), le bromure de stéaroyltriméthylammonium (STAB), le chlorure de benzalkonium (chlorure d'alkyldiméthylbenzylammonium), le bromure de N-cétylpyridinium (bromure de N-hexadécylpyridinium), le chlorure de N-cétylpyridinium (chlorure de N-hexadécylpyridinium), le chlorure de benzyl-diméthyltétradécylammonium, le chlorure de benzyldiméthyl-hexadécylammonium et une association de deux ou plus de deux quelconques d'entre eux.

**6.** Méthode suivant l'une quelconque des revendications précédentes, dans laquelle la cellule bactérienne, la cellule de levure ou la cellule fongique est mise en contact avec la composition d'extraction de membrane à des températures de 4˚C à 40˚C.

**7.** Méthode suivant l'une quelconque des revendications précédentes, dans laquelle la cellule bactérienne, la cellule de levure ou la cellule fongique est mise en contact avec la composition d'extraction de membrane à un pH de 2,0 à 11,0.

**8.** Méthode pour sélectionner des cellules mutées ou cellules transformées produisant des taux élevés d'une POI recombinante intracellulaire soluble ou associée aux membranes, comprenant les étapes consistant à :

(a) faire croître les cellules mutées à 30˚C ;
(b) mettre en incubation les cellules mutées ou cellules transformées avec la composition d'extraction de membrane de la manière définie dans une méthode de l'une quelconque des revendications précédentes ;
(c) recueillir le milieu dépourvu de cellules ;
(d) tester le milieu dépourvu de cellules pour des taux élevés de la POI recombinante intracellulaire ;
de telle sorte que la présence de la POI recombinante intracellulaire dans le milieu dépourvu de cellules soit une indication que la POI recombinante intracellulaire a été libérée, dans laquelle la cellule mutée ou transformée est une cellule bactérienne, une cellule de levure ou une cellule fongique et dans laquelle la POI recombinante est une enzyme hexose-oxydase (HOX).

**9.** Utilisation d'une composition d'extraction de membrane comprenant un composé d'ammonium quaternaire pour extraire une POI recombinante intracellulaire soluble ou associée aux membranes d'une cellule bactérienne, d'une cellule de levure ou d'une cellule fongique, dans laquelle la composition d'extraction de membrane comprend un composé d'ammonium quaternaire à une concentration de 0,05 % à 0,6 % en poids et est mise en contact avec la cellule bactérienne, la cellule de levure ou la cellule fongique, dans laquelle la POI recombinante libérée est recueillie à partir de la composition d'extraction de membrane et dans laquelle la POI recombinante est une enzyme hexose-oxydase (HOX).

**10.** Méthode suivant l'une quelconque des revendications précédentes, dans laquelle l'enzyme hexose-oxydase (HOX) comprend la séquence d'aminoacides indiquée sur la figure 6 (SEQ ID N˚ 22).

**11.** Méthode suivant la revendication 10, dans laquelle l'enzyme hexose-oxydase (HOX) est codée par une séquence de nucléotides indiquée sur la figure 6 (SEQ ID N˚ 22).

**12.** Méthode suivant la revendication 10 ou la revendication 11, dans laquelle l'enzyme hexose-oxydase (HOX) est codée par une séquence de nucléotides apte à l'hybridation à la séquence de nucléotides indiquée sur la figure 6 (SEQ ID N˚ 22).

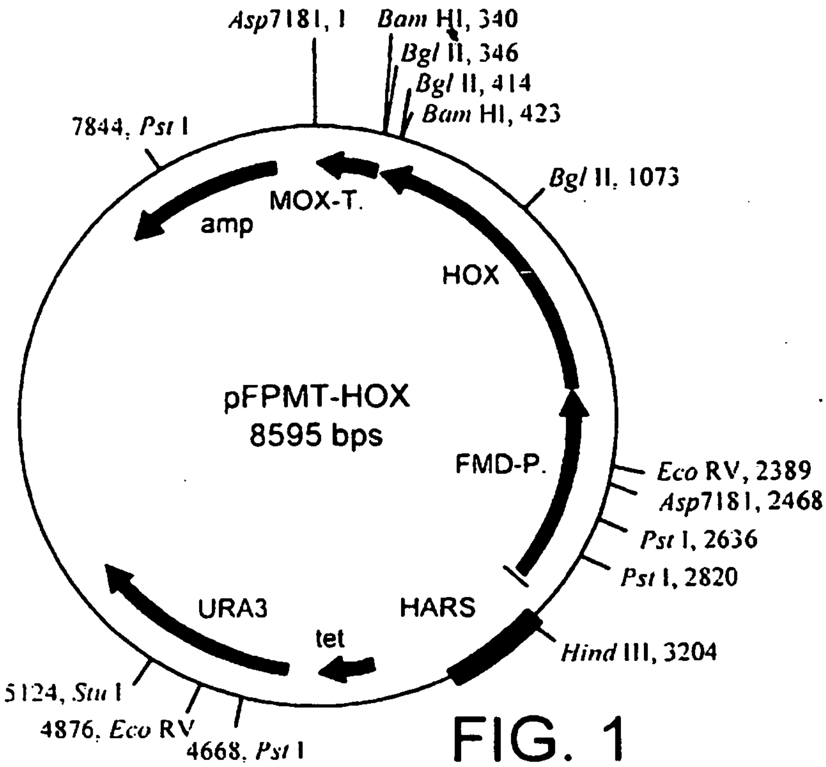
Asp718I, 1
Bam HI, 340
Bgl II, 346
Bgl II, 414
Bam HI, 423
7844, Pst I
Bgl II, 1073
MOX-T.
amp
HOX
pFPMT-HOX
8595 bps
FMD-P.
Eco RV, 2389
Asp718I, 2468
Pst I, 2636
Pst I, 2820
URA3
HARS
tet
Hind III, 3204
5124, Stu I
4876, Eco RV
4668, Pst I

FIG. 1

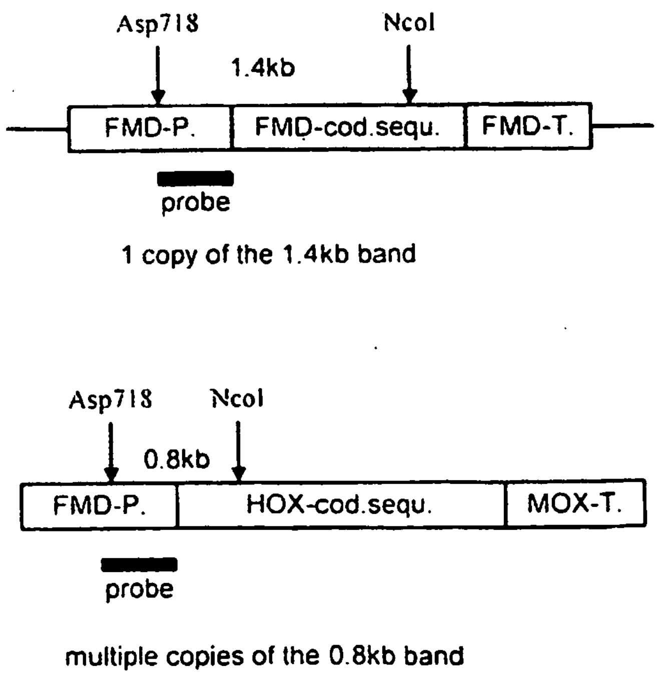
Asp718
NcoI
1.4kb
FMD-P.
FMD-cod.sequ.
FMD-T.
probe
1 copy of the 1.4kb band
Asp718
NcoI
0.8kb
FMD-P.
HOX-cod.sequ.
MOX-T.
probe
multiple copies of the 0.8kb band

FIG. 2A

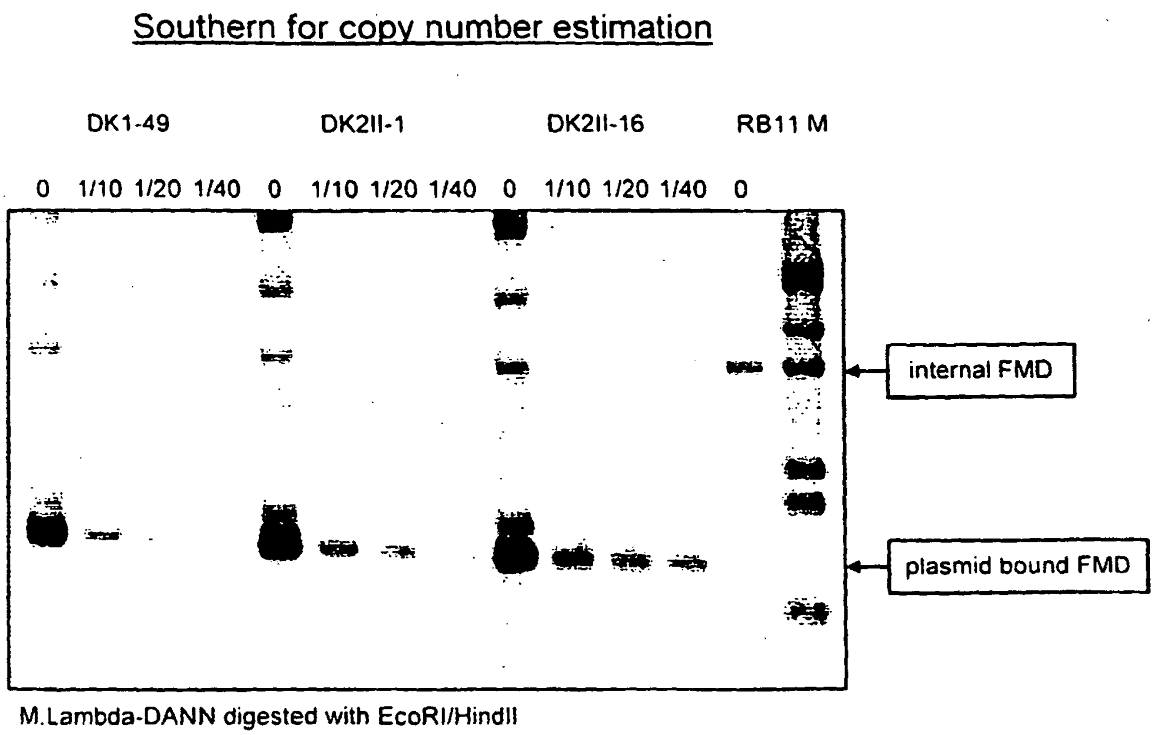
Southern for copy number estimation
DK1-49
DK2II-1
DK2II-16
RB11 M
0 1/10 1/20 1/40 0 1/10 1/20 1/40 0 1/10 1/20 1/40 0
internal FMD
plasmid bound FMD
M.Lambda-DANN digested with EcoRI/HindII

FIG. 2B

# FIG. 3A

Supernatants from glycerol-fermentation of strain DK8-27 KanII3-mut25

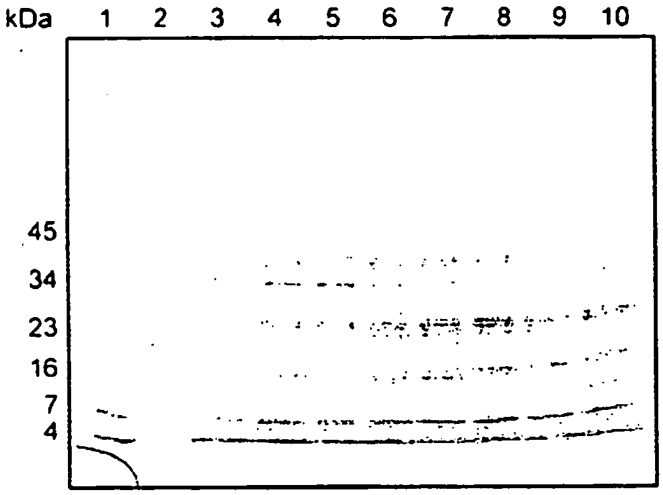


lane 1: Marker
lane 2: HOX-standard (0.03U/ml; 18µl)
lane 3: supernatant from probe3 (18µl)
lane 4: " probe4 (18µl)
lane 5: " probe5 (18µl)
lane 6: " probe6 (18µl)
lane 7: " probe7 (18µl)
lane 8: " probe8 (18µl)
lane 9: " probe9 (18µl)
lane 10 " probe10 (18µl)

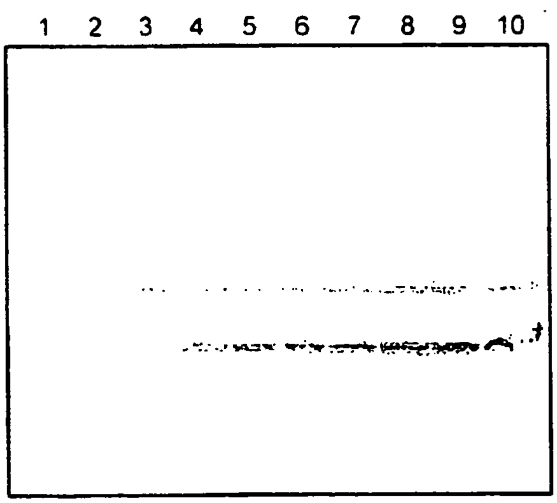


Same positions of probes as above

The Western-blot shows (in accordance to the measurements) that there is no real incease in HOX production during the last 50 hours of the fermentation

# FIG. 3B

D-Hox-1

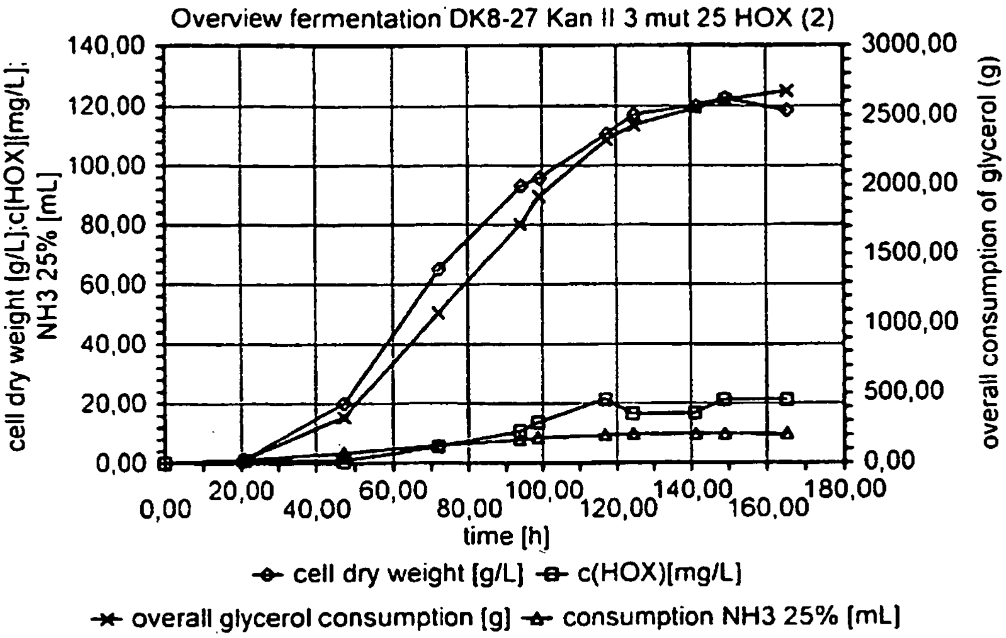
Overview fermentation DK8-27 Kan II 3 mut 25 HOX (2)
cell dry weight [g/L];c[HOX)][mg/L]; NH3 25% [mL]
overall consumption of glycerol (g)
140,00
120,00
100,00
80,00
60,00
40,00
20,00
0,00
3000,00
2500,00
2000,00
1500,00
1000,00
500,00
0,00
0,00
20,00
40,00
60,00
80,00
100,00
120,00
140,00
160,00
180,00
time [h]
cell dry weight [g/L]
c(HOX)[mg/L]
overall glycerol consumption [g]
consumption NH3 25% [mL]

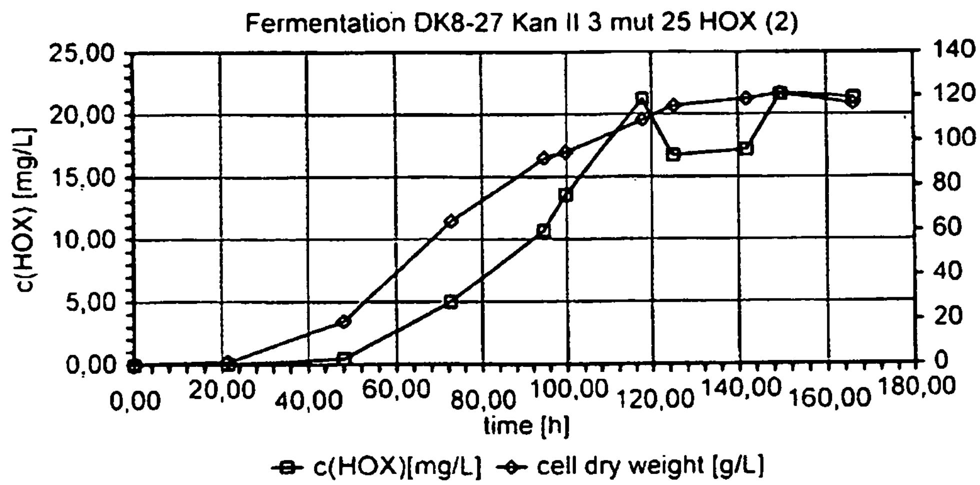
Fermentation DK8-27 Kan II 3 mut 25 HOX (2)
c(HOX) [mg/L]
cdw (g/L)
25,00
20,00
15,00
10,00
5,00
0,00
140
120
100
80
60
40
20
0
0,00
20,00
40,00
60,00
80,00
100,00
120,00
140,00
160,00
180,00
time [h]
c(HOX)[mg/L]
cell dry weight [g/L]

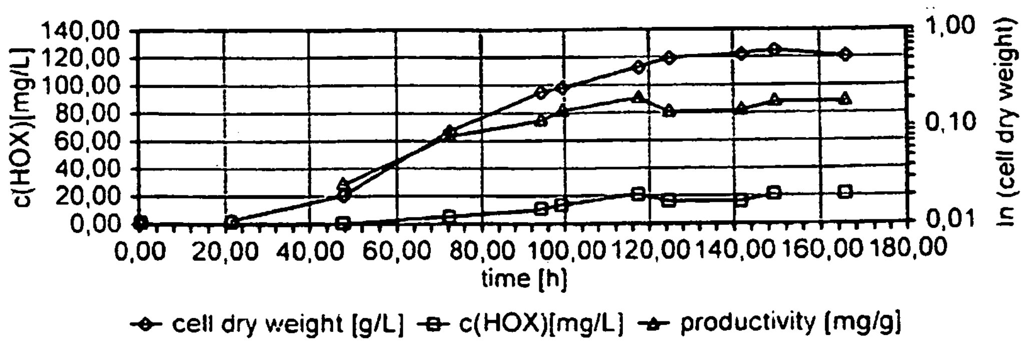
c(HOX)[mg/L]
ln (cell dry weight)
140,00
120,00
100,00
80,00
60,00
40,00
20,00
0,00
1,00
0,10
0,01
0,00
20,00
40,00
60,00
80,00
100,00
120,00
140,00
160,00
180,00
time [h]
cell dry weight [g/L]
c(HOX)[mg/L]
productivity [mg/g]

FIG. 4

# FIG. 5

**hox1A**
ACTCCATGGCTACTTTGCCACAAAAGGACCCAGGTTACATTGTTATTGACG
TCAACGCTGG(SEQ ID No 2)

**hox2B**
CGAAATCGATGTTGGTACCAATCCATCTTCTGTTGAAACCTTGCTTCATGG
ATGGCAATCTTGGGTCAGGCTTGTCTGGAGTACCAGCGTTGACGTCAATAA
CAATG(SEQ ID No 3)

**hox3a**
GATTGGTACCAACATCGATTTCGTTTACGTCGTTTACACTCCACAAGGTGC
TTGTACTGCTTTGGACAGAGCTATGGAAAAGTGTTCTCCAGGTACCGTCAG
AATC(SEQ ID No 4)

**hox4b**
TTCAACCAAACCAGTAACGTTGATAATAGCCTTGACACATTCGTCGAAAAC
GAAGTCTTCGTAACAGTGACCACCAGAAACGATTCTGACGGTACCTGGAGA
ACAC(SEQ ID No5)

**hox5a**
ATCAACGTTACTGGTTTGGTTGAATCTGGTTACGACGACGATAGAGGTTAC
TTCGTCTCTTCCGGTGACACCAACTGGGGTTCCTTCAAGACCTTGTTCAGA
GACCACGGTAGAGTTTTG(SEQ ID No6)

**hox6b**
CAAACCGTGCAATCTGGCCAAAATACCGTCACCTCCACCGACAATGTGACC
ACCCAAACCGACGGAGTAACAGGAACCACCTGGCAAAACTCTACCGTGGTC
TCTGAAC(SEQ ID No 7)

**hox7a**
TTTGGCCAGATTGCACGGTTTGCCAGTCGATTGGTTATCCGGTGTTGAAGT
TGTCGTTAAGCCAGTCTTGACCGAAGACTCTGTTCTTAAGTACGTTCACAA
GGATTCC(SEQ ID No 8)

**hox8b**
GGCAAATCCTTGAAGTAGTATTTGGTGATAATACCGAAGTTACCTCCACCT
CCACCAGTGTGAGCCCAAAACAACTCACCGTCGTTACCTTCGGAATCCTTG
TGAACGTACTTAAG(SEQ ID No 9)

**hox9a**
CAAATACTACTTCAAGGATTTGCCAATGTCTCCAAGAGGTGTCATCGCTTC
TAACTTACACTTCTCTTGGGACGGTTTCACTAGAGATGCCTTGCAAGATTT
GTTGACTAAGTACTTC(SEQ ID No 10)

**hox10b**
GGAGGTATACAAGTACATAACAAACTCTTCAGCTGCTTGGTGGAAGATTTG
GAACTTACCAACAGTATTCTTCCAATCACATCTAGCCAACTTGAAGTACTT
AGTCAACAAATCTTGC(SEQ ID No 11)

**primer 1**
ATCTTCCATCAGGCAGCTGAAGAGTTTGTTATGTACTTGTATACATCCTAC
TCTAACGACGCCGAGAGAGAAGTTGCCCAAGACAGACACTATCAT
(SEQ ID No 12)

**primer 2**
GAAAGGAGCCCAACCAGCATGACCACCAAGAGCTTTGGTAGGCTCGCATGT
TTTGTAGATCTGTTCAATGTCAGCCTCCAAATGATAGTGTCTGTCTTGGGC
(SEQ ID No 13)

**primer 3**
GCTGGTTGGGCTCCTTTCCCTGTTAGACCTAGACCTAGACACACATCCAAG
ACTTCTTATATGCATGACGAGACTATGGACTACCCTTTC
(SEQ ID No 14)

**primer 4**
AATCTGGAAGTCTGGAAAGTCCTTGATCATGTAAGCAGACTTGTACTTACC
TCTCTGATTAGGACCGGAACCGTTGATAGTCTCAGTCAAAGCGTAGAAAGG
GTAGTCCATAGTCTCGTC(SEQ ID No 15)

**primer 5**
GACTTTCCAGACTTCCAGATTGATGTTATCTGGAAATACCTTACTGAGGTT
CCTGACGGTTTGACTAGTGCCGAAATGAAGGATGCTCTTCTTCAGGTTGAT
ATGTTC(SEQ ID No 16)

**primer 6**
CTTGTCTTCTTCCTGCCAGTATGTCTGGTACTGCAGTTTGATGATGTACTC
TCTCTGAGCAACTGCAGTAGCATCCCAAACAACCTTGTGAATCTCACCACC
GAACATATCAACCTGAAGAAGAGC(SEQ ID No 17)

**primer 7**
ACATACTGGCAGGAAGAAGACAAGGATGCAGTTAACTTGAAGTGGATTAGA
GACTTTTACGAGGAGATGTATGAGCCTTATGGTGGTGTTCCAGACCCTAAC
ACTCAG(SEQ ID No 18)

**primer 8**
GGCACCATACTTACCGTTCTTCCAGTTGTTCAAGTCAACATCAGGGTAGTT
GAAGTAGCATCCCTCAAAAACACCTTTACCACTCTCAACCTGAGTGTTAGG
GTCTGGAAC(SEQ ID No 19)

**primer 9**
AAGAACGGTAAGTATGGTGCCTTGGAACTTTACTTTTTGGGTAACCTGAAC
AGATTGATCAAGGCCAAATGGTTGTGGGATCCTAACGAGATCTTCACAAAC
AAACAGTCTATCCCT(SEQ ID No 20)

**primer 10**
GAATTCCGCGGCCGCCTACTATTTAGTCTGCTTAGGCTCCTTAAGAGGTTT
AGTAGGGATAGACTGTTTGTTTGTGAA(SEQ ID No 21)

FIG. 5CONT'D

# FIG. 6

## (SEQ ID No 22)

```
Molecule Name:     hoxpic                       1644 bps DNA Linear
Sequence Printed: 1-1644 (Full)                 Date Printed  04 Jun 1999
Description:

  1  ATG GCT ACT TTG CCA CAA AAG GAC CCA GGT TAC ATT GTT ATT
      M   A   T   L   P   Q   K   D   P   G   Y   I   V   I

 43  GAC GTC AAC GCT GGT ACT CCA GAC AAG CCT GAC CCA AGA TTG
      D   V   N   A   G   T   P   D   K   P   D   P   R   L

 85  CCA TCC ATG AAG CAA GGT TTC AAC AGA AGA TGG ATT GGT ACC
      P   S   M   K   Q   G   F   N   R   R   W   I   G   T

127  AAC ATC GAT TTC GTT TAC GTC GTT TAC ACT CCA CAA GGT GCT
      N   I   D   F   V   Y   V   V   Y   T   P   Q   G   A

169  TGT ACT GCT TTG GAC AGA GCT ATG GAA AAG TGT TCT CCA GGT
      C   T   A   L   D   R   A   M   E   K   C   S   P   G

211  ACC GTC AGA ATC GTT TCT GGT GGT CAC TGT TAC GAA GAC TTC
      T   V   R   I   V   S   G   G   H   C   Y   E   D   F

253  GTT TTC GAC GAA TGT GTC AAG GCT ATT ATC AAC GTT ACT GGT
      V   F   D   E   C   V   K   A   I   I   N   V   T   G

295  TTG GTT GAA TCT GGT TAC GAC GAC GAT AGA GGT TAC TTC GTC
      L   V   E   S   G   Y   D   D   D   R   G   Y   F   V

337  TCT TCC GGT GAC ACC AAC TGG GGT TCC TTC AAG ACC TTG TTC
      S   S   G   D   T   N   W   G   S   F   K   T   L   F

379  AGA GAC CAC GGT AGA GTT TTG CCA GGT GGT TCC TGT TAC TCC
      R   D   H   G   R   V   L   P   G   G   S   C   Y   S

421  GTC GGT TTG GGT GGT CAC ATT GTC GGT GGA GGT GAC GGT ATT
      V   G   L   G   G   H   I   V   G   G   G   D   G   I

463  TTG GCC AGA TTG CAC GGT TTG CCA GTC GAT TGG TTA TCC GGT
      L   A   R   L   H   G   L   P   V   D   W   L   S   G

505  GTT GAA GTT GTC GTT AAG CCA GTC TTG ACC GAA GAC TCT GTT
      V   E   V   V   V   K   P   V   L   T   E   D   S   V

547  CTT AAG TAC GTT CAC AAG GAT TCC GAA GGT AAC GAC GGT GAG
      L   K   Y   V   H   K   D   S   E   G   N   D   G   E

589  TTG TTT TGG GCT CAC ACT GGT GGA GGT GGA GGT AAC TTC GGT
      L   F   W   A   H   T   G   G   G   G   G   N   F   G

631  ATT ATC ACC AAA TAC TAC TTC AAG GAT TTG CCA ATG TCT CCA
      I   I   T   K   Y   Y   F   K   D   L   P   M   S   P

673  AGA GGT GTC ATC GCT TCT AAC TTA CAC TTC TCT TGG GAC GGT
      R   G   V   I   A   S   N   L   H   F   S   W   D   G

715  TTC ACT AGA GAT GCC TTG CAA GAT TTG TTG ACT AAG TAC TTC
      F   T   R   D   A   L   Q   D   L   L   T   K   Y   F

757  AAG TTG GCT AGA TGT GAT TGG AAG AAT ACT GTT GGT AAG TTC
      K   L   A   R   C   D   W   K   N   T   V   G   K   F
```

```
799   CAA ATC TTC CAC CAA GCA GCT GAA GAG TTT GTT ATG TAC TTG
       Q   I   F   H   Q   A   A   E   E   F   V   M   Y   L

841   TAT ACA TCC TAC TCT AAC GAC GCC GAG AGA GAA GTT GCC CAA
       Y   T   S   Y   S   N   D   A   E   R   E   V   A   Q

883   GAC AGA CAC TAT CAT TTG GAG GCT GAC ATT GAA CAG ATC TAC
       D   R   H   Y   H   L   E   A   D   I   E   Q   I   Y

925   AAA ACA TGC GAG CCT ACC AAA GCT CTT GGT GGT CAT GCT GGT
       K   T   C   E   P   T   K   A   L   G   G   H   A   G

967   TGG GCT CCT TTC CCT GTT AGA CCT AGA AAG AGA CAC ACA TCC
       W   A   P   F   P   V   R   P   R   K   R   H   T   S

1009  AAG ACT TCT TAT ATG CAT GAC GAG ACT ATG GAC TAC CCT TTC
       K   T   S   Y   M   H   D   E   T   M   D   Y   P   F

1051  TAC GCT TTG ACT GAG ACT ATC AAC GGT TCC GGT CCT AAT CAG
       Y   A   L   T   E   T   I   N   G   S   G   P   N   Q

1093  AGA GGT AAG TAC AAG TCT GCT TAC ATG ATC AAG GAC TTT CCA
       R   G   K   Y   K   S   A   Y   M   I   K   D   F   P

1135  GAC TTC CAG ATT GAT GTT ATC TGG AAA TAC CTT ACT GAG GTT
       D   F   Q   I   D   V   I   W   K   Y   L   T   E   V

1177  CCT GAC GGT TTG ACT AGT GCC GAA ATG AAG GAT GCT CTT CTT
       P   D   G   L   T   S   A   E   M   K   D   A   L   L

1219  CAG GTT GAT ATG TTC GGT GGT GAG ATT CAC AAG GTT GTT TGG
       Q   V   D   M   F   G   G   E   I   H   K   V   V   W

1261  GAT GCT ACT GCA GTT GCT CAG AGA GAG TAC ATC ATC AAA CTG
       D   A   T   A   V   A   Q   R   E   Y   I   I   K   L

1303  CAG TAC CAG ACA TAC TGG CAG GAA GAA GAC AAG GAT GCA GTT
       Q   Y   Q   T   Y   W   Q   E   E   D   K   D   A   V

1345  AAC TTG AAG TGG ATT AGA GAC TTT TAC GAG GAG ATG TAT GAG
       N   L   K   W   I   R   D   F   Y   E   E   M   Y   E

1387  CCT TAT GGT GGT GTT CCA GAC CCT AAC ACT CAG GTT GAG AGT
       P   Y   G   G   V   P   D   P   N   T   Q   V   E   S

1429  GGT AAA GGT GTT TTT GAG GGA TGC TAC TTC AAC TAC CCT GAT
       G   K   G   V   F   E   G   C   Y   F   N   Y   P   D

1471  GTT GAC TTG AAC AAC TGG AAG AAC GGT AAG TAT GGT GCC TTG
       V   D   L   N   N   W   K   N   G   K   Y   G   A   L

1513  GAA CTT TAC TTT TTG GGT AAC CTG AAC AGA TTG ATC AAG GCC
       E   L   Y   F   L   G   N   L   N   R   L   I   K   A

1555  AAA TGG TTG TGG GAT CCT AAC GAG ATC TTC ACA AAC AAA CAG
       K   W   L   W   D   P   N   E   I   F   T   N   K   Q

1597  TCT ATC CCT ACT AAA CCT CTT AAG GAG CCT AAG CAG ACT AAA
       S   I   P   T   K   P   L   K   E   P   K   Q   T   K

1639  TAG TAG
```

FIG. 6CONT'D

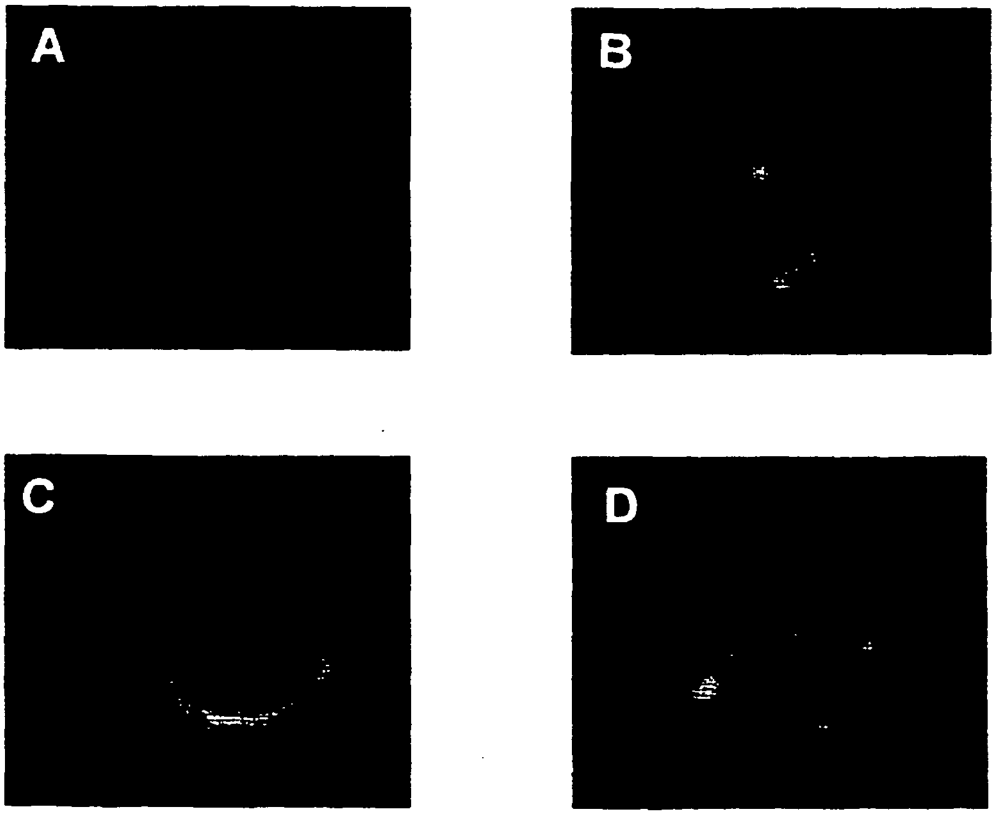


FIG. 7

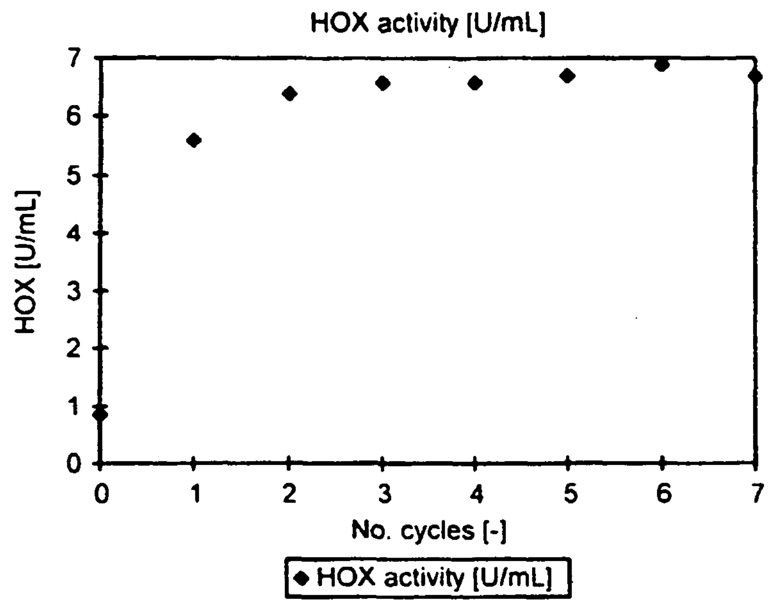
HOX activity [U/mL]
HOX [U/mL]
0
1
2
3
4
5
6
7
No. cycles [-]
◆ HOX activity [U/mL]

FIG. 8

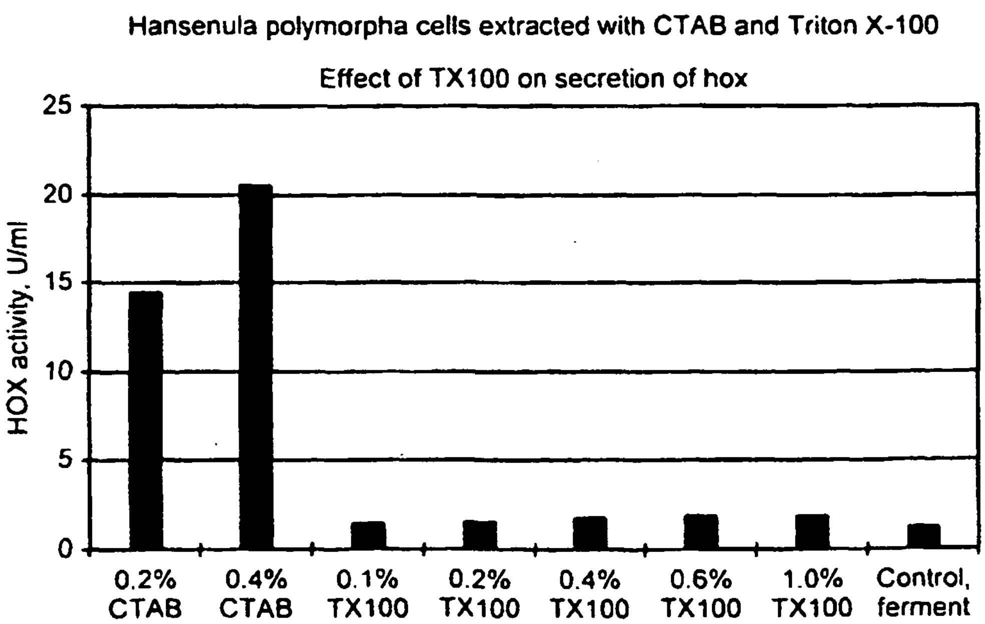
Hansenula polymorpha cells extracted with CTAB and Triton X-100
Effect of TX100 on secretion of hox
HOX activity, U/ml
0
5
10
15
20
25
0.2% CTAB
0.4% CTAB
0.1% TX100
0.2% TX100
0.4% TX100
0.6% TX100
1.0% TX100
Control, ferment

FIG. 9

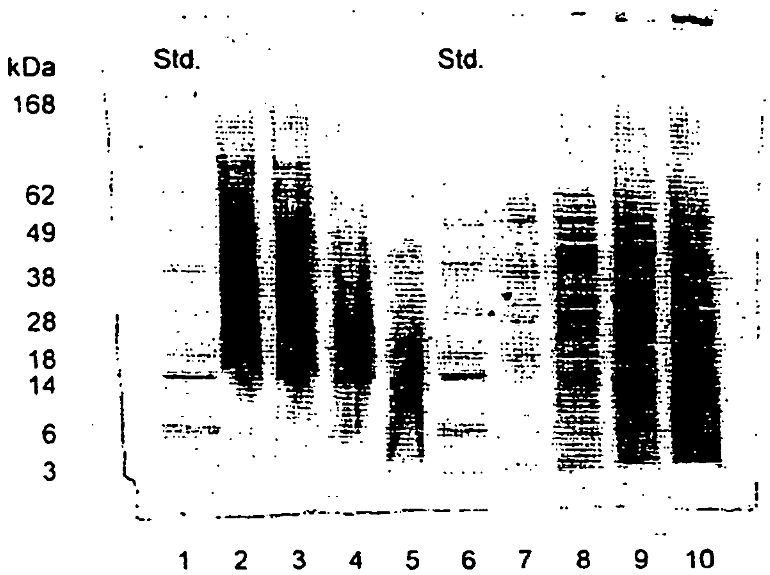


FIG. 10A

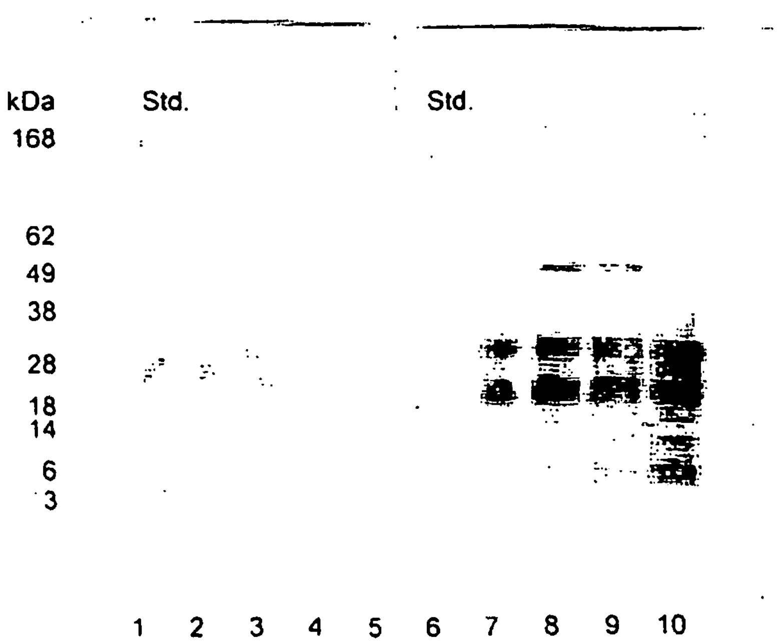


FIG. 10B

FIG. 11A

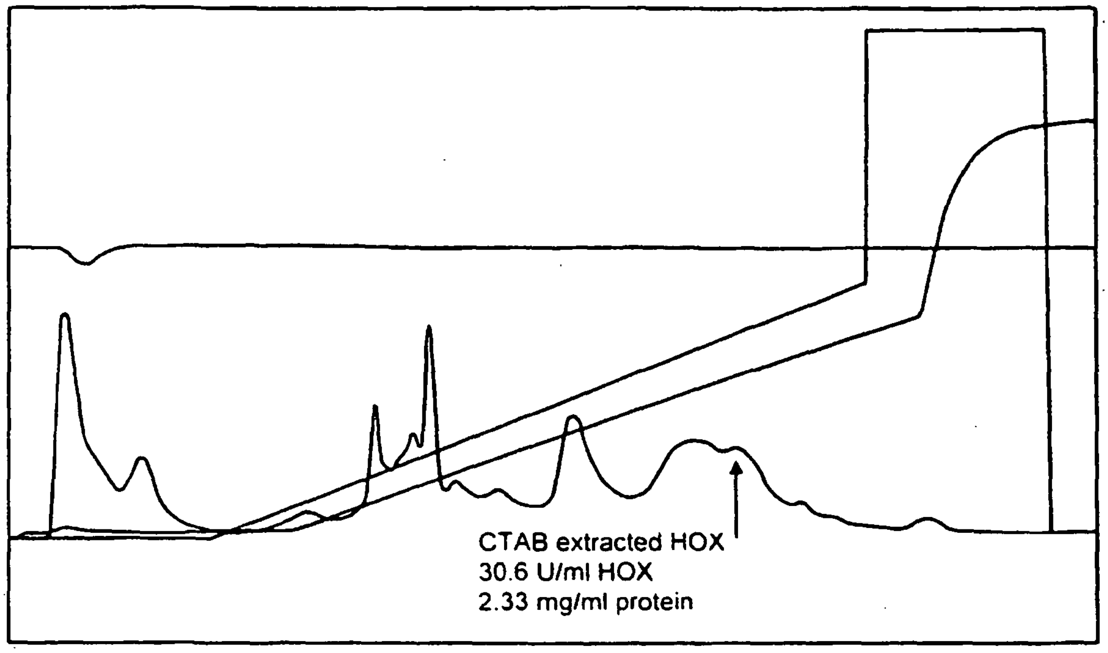


FIG. 11B

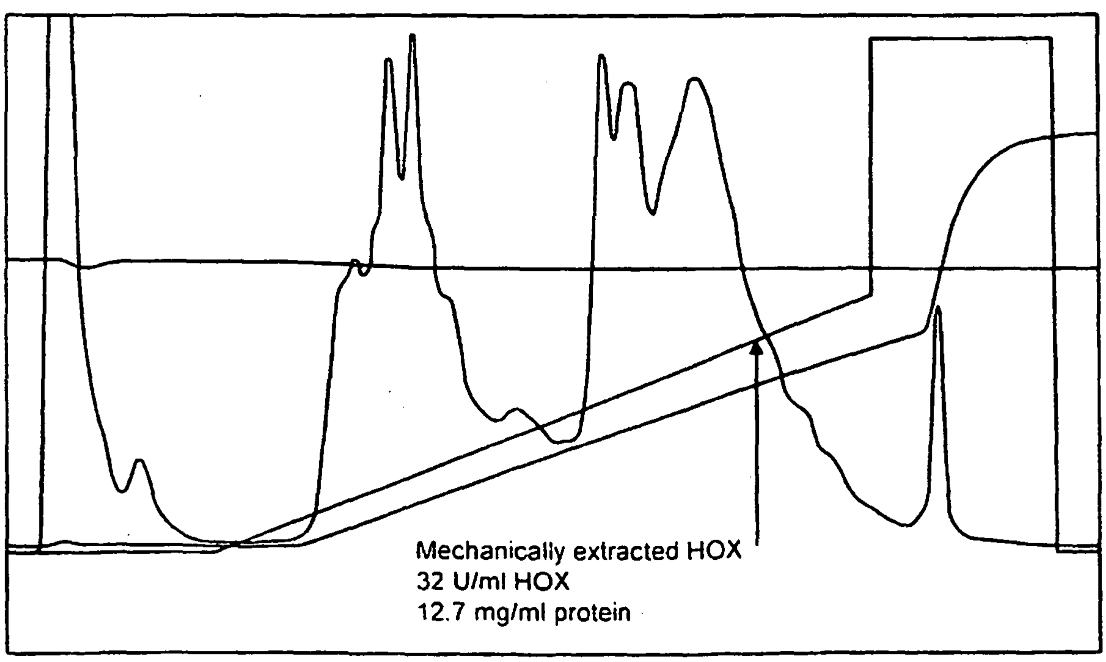

# FIG. 12A

western blot (WB 33)

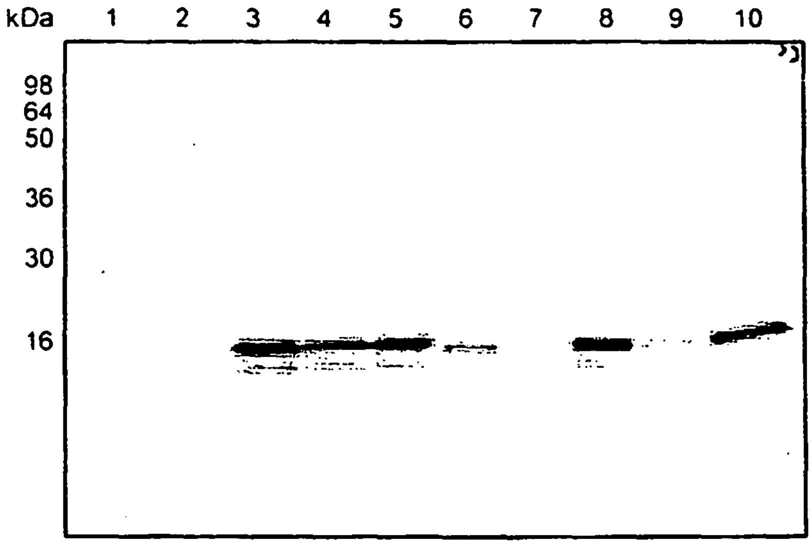


# FIG. 12B

collidal coomassie (Coo 2)

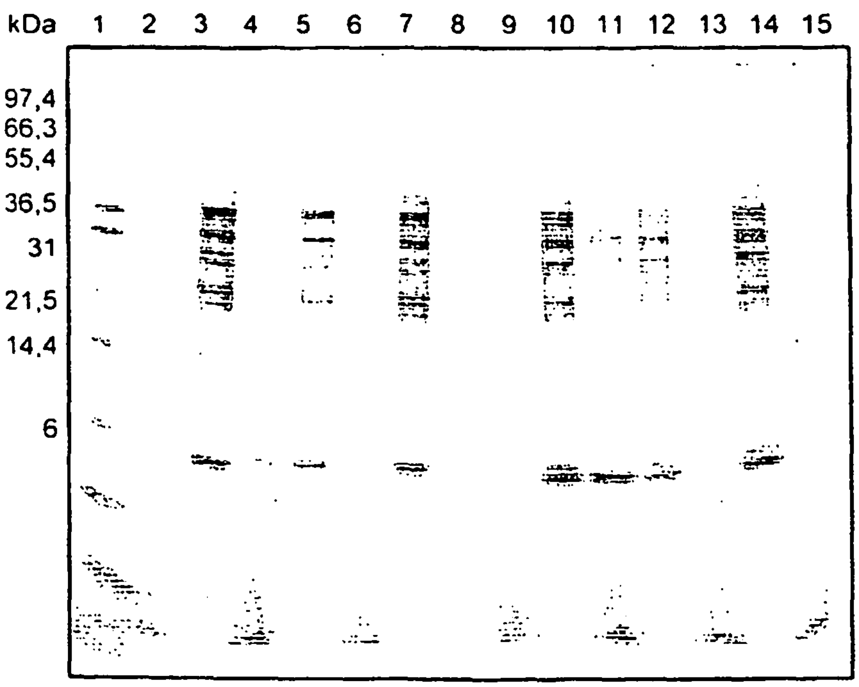

# FIG. 13A

western blot (WB 34)

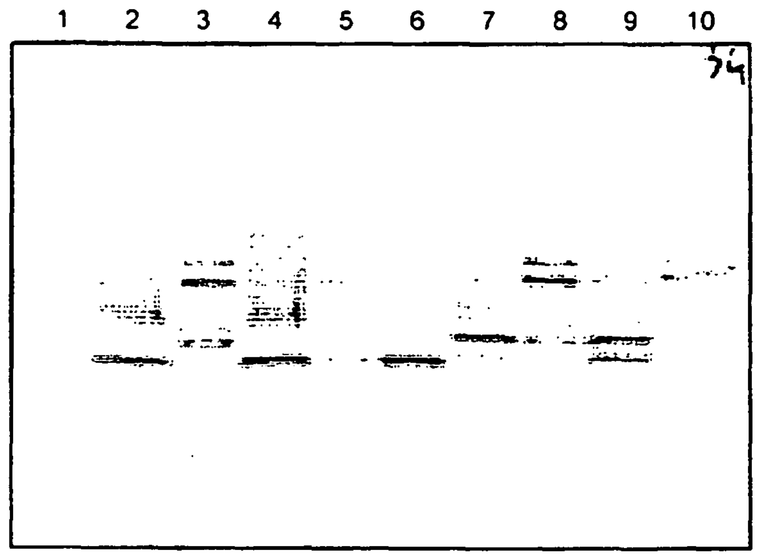


# FIG. 13B

collidal coomassie (Coo 3)

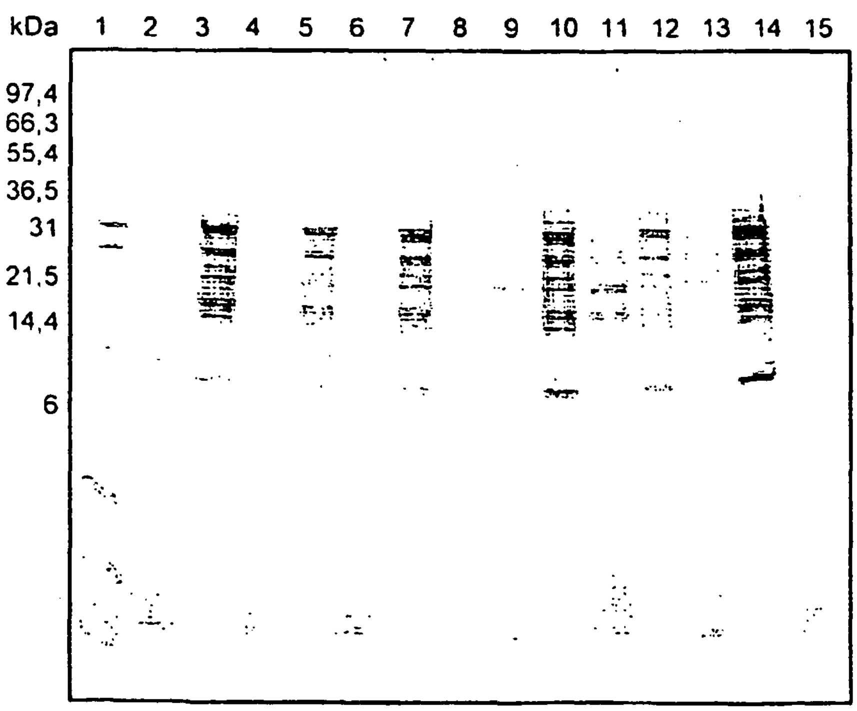

FIG. 14A

western blot (WB 35)

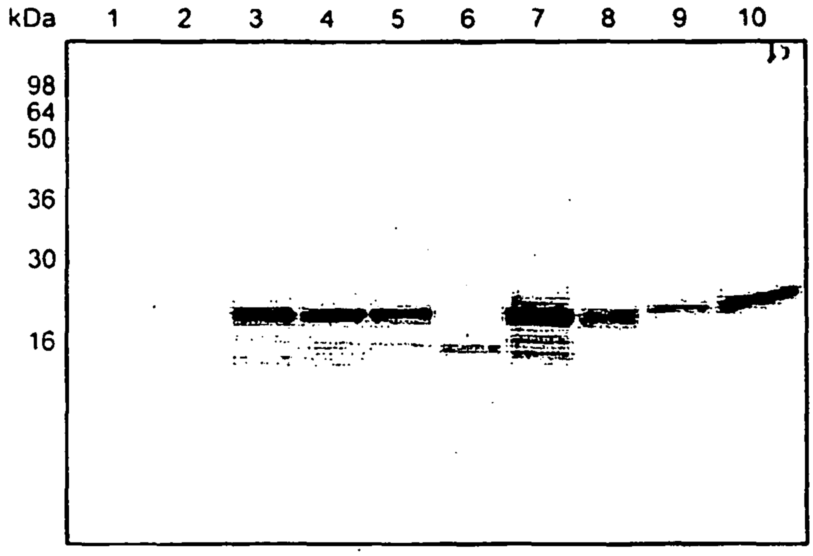


FIG. 14B

collidal coomassie (Coo 4)

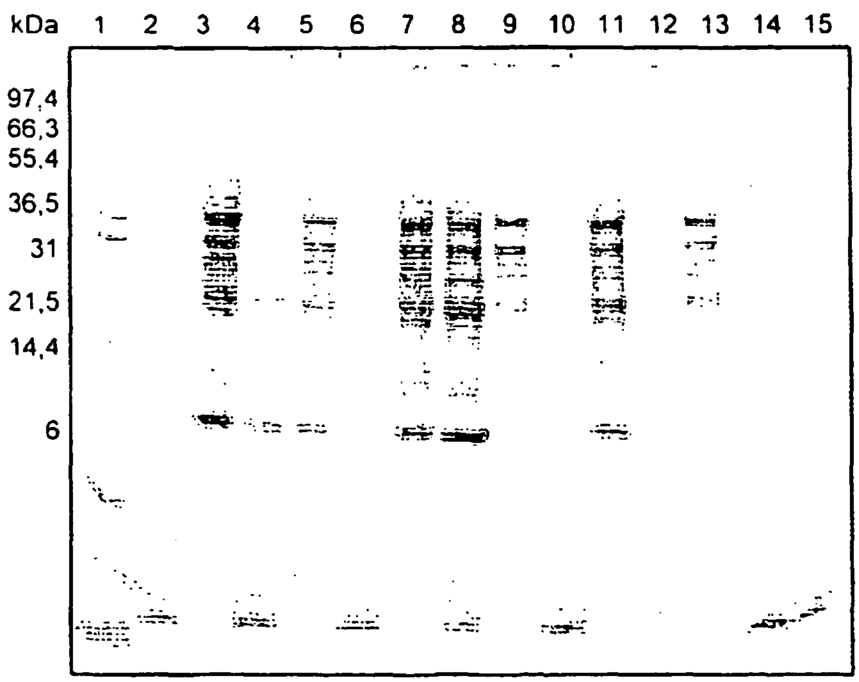

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5124256 A, Crahay
- US 1992 A
- US 3801461 A, Miyake and Shiosaka
- US 1974 A
- US 4683293 A, Craig
- US 1987 A
- WO 9639851 A
- EP 0833563 B
- WO 9640935 A
- WO 9813478 A


### Non-patent literature cited in the description


- **GOWDA et al.** *Enzyme Microb. Technol,* 1991, vol. 13, 154-157
- **ALAMÄE ; JÄRVISTE.** *Journal of Microbiological Methods,* 1995, vol. 22, 193-205
- **AHLSTROM ; EDEBO.** *PEMS Microbiology Letters,* 1994, vol. 119, 7-12
- **ISHAQ et al.** *Biotechniques,* 1990, vol. 9 (1), 19-2022, 24
- **KAMBOURIS et al.** *FEMS Immunol Med Microbiol,* 1999, vol. 25 (3), 255-64
- **KUIPERS et al.** *Ann Rheum Dis,* 1999, vol. 58 (2), 103-8
- **VELEGRAKI et al.** *Med Mycol,* 1999, vol. 37 (1), 69-73
- **WHITE et al.** *Med Mycol,* 1998, vol. 36 (5), 299-303
- **WOODHEAD et al.** *Mol Biotechnol,* 1998, vol. 9 (3), 243-6
- **MITO ; DETSCHART.** *Parasitol Res,* 1998, vol. 84 (7), 596-7
- **ZHANG et al.** *J Virol Methods,* 1998, vol. 71 (1), 45-50
- **REINEKE et al.** *Insect Mol Biol,* 1998, vol. 7 (1), 95-9
- **DEVEREUX et al.** *Nucleic Acids Research,* 1984, vol. 12, 387
- **AUSUBEL et al.** J.MOL.BIOL. 1999
- **ATSCHUL et al.** *J. Mol. Biol.,* 1990, 403-410
- **AUSUBEL et al.** *J.MOL.BIOL.,* 1999, 7-587-60
- **BERGER ; KIMMEL.** Guide to Molecular Cloning Techniques, Methods in Enzymology. Academic Press, 1987, vol. 152
- **MURRAY E et al.** *Nuc Acids Res,* 1989, vol. 17, 477-508
- **BOLLAG, D.M. ; EDELSTEIN, S.J.** *Protein Methods.,* 1991
- **GOWDA, L.R. ; BACHHAWAT, N. ; BHAT, S.G.** Permeabilization of baker's yeast by cetyltrimethylammonium bromide for intracellular enzyme catalysis. *Enzyme Microb. Technol.,* 1991, vol. 13, 154-157
- **HANSEN, O.C. ; STOUGAARD, P.** Hexose oxidase from the red alga Chondrus crispus: purification, molecular cloning, and expression in Pichia pastoris. *J. Biol. Chem.,* 1997, vol. 272, 11581-11587
- **JOSHI, M.S. ; GOWDA, L.R. ; BHAT, S.G.** Permeabilization of yeast cells (Kluyveromyces fragilis) to lactose by cetyltrimethylammonium bromide. *Biotechnol. Lett.,* 1987, vol. 9, 549-554
- **JOSHI, M.S. ; GOWDA, L.R. ; KATWA, L.C. ; BHAT, S.G.** Permeabilization of yeast cells (Kluyveromyces fragilis) to lactose by digitonin. *Enzyme Microb. Technol.,* 1989, vol. 11, 439-443
- **HAGEN, I.M. ; HYAM, J.S.** *J. Cell Sci.,* 1988, vol. 89, 343-357
- **HUNKAPILLER, M.W. ; LUJAN, U. ; OSTRANDER, F. ; HOOD, L.E.** Isolation of proteins from polyacrylamide gels for amino acid sequence analysis. *Methods in Enzymology,* 1983, vol. 91, 227-236
- **KING, A.T. ; DAVEY, M.R. ; MELLOR, I.R ; MULLIGAN, B.J. ; LOWE, K.C.** Surfactant effects on yeast cells. *Enzyme Microb. Technol.,* 1991, vol. 13, 148-153
- Chemical permeabilization of cells for intracellular product release. **NAGLAK, T.J. ; HETTWER, D.J. ; WANG, H.Y.** Separation processes in biotechnology. M. Dekker, 1990, vol. 9
- **POULSEN, C.H. ; HØSTRUP, P.B.** Purification and characterization of a hexose oxidase with excellent strengthening effects in bread. *Cereal Chem.,* 1998, vol. 75, 51-57
- **SCHLEIF, R.F. ; WENSINK, P.C.** Practical Methods in Molecular Biology. Springer-Verlag, 1981

- **SEKHAR, S. ; BHAT, N. ; BHAT, S.G.** Preparation of detergent permeabilized Bakers' yeast whole cell catalase. *Process Biochem.,* 1999, vol. 34, 349-354
- **SULLIVAN, J. D. ; IKAWA, M.** Purification and characterization of hexose oxidase from the red alga chondrus crispus. *Biochem. Biophys. Acta,* 1973, vol. 309, 11-22